# EUROPEAN PATENT SPECIFICATION

(11) **EP 1 957 526 B1**
(45) Date of publication and mention of the grant of the patent: **26.06.2013**
(21) Application number: 05819136.2
(22) Date of filing: 20.12.2005
(51) Int. Cl.: C07K 14/47

(54) **MOLECULES INHIBITION INTERCELLULAR ADHESION**
DIE INTERZELLULÄRE ADHÄSION INHIBIERENDE MOLEKÜLE
MOLECULES INHIBANT L'ADHESION INTERCELLULAIRE

(30) Priority: 20.12.2004 KR 20040108909
(43) Date of publication of application: 20.08.2008
(73) Proprietor: Isu Abxis Co., Ltd., Seodaemun-gu, Seoul 120-652 (KR)
(72) Inventor: KIM, Myung Kyung, Bethesda, MD 20817 (US); CHUNG, Jay Hang, Bethesda, MD 20817 (US)
(74) Representative: Grünecker, Kinkeldey, Stockmair & Schwanhäusser
(86) International application number: PCT/KR2005/004398
(87) International publication number: WO 2006/068398

(56) References cited:
- EP-A- 0 972 524
- EP-A- 1 059 533
- EP-A- 1 394 274
- ISHIKAWA J ET AL: "Molecular cloning and chromosomal mapping of a bone marrow stromal cell surface gene, BST2, that may be involved in pre-B-cell growth" GENOMICS, ACADEMIC PRESS, SAN DIEGO, US, vol. 26, no. 3, 10 April 1995 (1995-04-10), pages 527-534, XP004828668 ISSN: 0888-7543
- DATABASE GENPEPT [Online] 08 February 2003 ISHIKAWA J. ET AL.: 'Molecular cloning and chromosomal mapping of a bone marrow stromal cell surface gene, BST2, that may be involved in pre-B-cell growth', XP003024121 Retrieved from NCBI Database accession no. (BAA05679) & GENOMICS vol. 26, no. 3, 1995, pages 527 - 534
- DATABASE GENPEPT [Online] 02 December 2004 STRAUSBERG R.L. ET AL.: 'Generation and initial analysis of more than 15,000 full-length human and mouse cDNA sequences', XP003024122 Retrieved from NCBI Database accession no. (AAH56638) & PROC. NATL. ACAD. SCI. USA vol. 99, no. 26, 2002, pages 16899 - 16903
- OHTOMO T. ET AL.: 'Molecular Cloning and Characterization of a Surface Antigen Preferentially Overexpressed on Multiple Myeloma Cells' BIOCHEM. BIOPHYS. RES. COMMUN. vol. 258, 1999, pages 583 - 591, XP002950705

## Description

### Technical Field

The present invention relates to molecules inhibiting intercellular adhesion during inflammation and the use of the same. More particularly, the present description relates to the Bst2 protein and fragments thereof inhibiting intercellular adhesion of cells participating in inflammation. The present invention is also concerned with a composition, comprising the same, and its use for preventing or treating inflammation-associated diseases, in particular asthma.

### Background Art

Inflammation is a normal response of the body to protect tissues from infection, injury or diseases. The inflammatory response begins with the production and release of chemical agents by cells in the affected tissues. The chemical agents cause redness, swelling, pain, heat and loss of function. Cells in inflamed tissues generate signals that recruit leukocytes to the site of inflammation. Leukocytes must adhere to endothelial cells to migrate from the bloodstream into the site of inflammation. Also, leukocytes should adhere to antigen-presenting cells to allow normal specific immune responses, and should finally adhere to suitable target cells to lyse pathogen-infected cells, cancer cells, or the like. The recruited leukocytes eliminate any infective or injurious agent and remove debris of damaged cells from the injured tissue.

The infiltrating leukocytes play critical roles in tissue regeneration and immune response in normal inflammation by engulfing invading microorganisms or dead cells. However, the infiltrating leukocytes cause serious or lethal status in pathological chronic inflammation. The abnormal recognition of self cells as non-self (foreign) or excess inflammation by sustained inflammatory responses causes a variety of inflammatory diseases including diabetes mellitus, atherosclerosis, cataract, reperfusion injury, infectious meningitis, rheumatoid arthritis, asthma, sepsis, inflammatory bowel disease and multiple sclerosis.

The interaction between leukocytes and endothelial cells is as follows.

Leukocytes have dual functions to act in a form circulating in the bloodstream or adhering to specific cells. In particular, adherent leukocytes interact with endothelial cells, stabilize intercellular adhesion with antigen-presenting cells or act as effector cells to migrate into inflammatory or infected sites. For normal specific immune response, leukocytes should adhere to antigen-presenting cells and should finally adhere to suitable target cells to lyse pathogen-infected cells, cancer cells, or the like. A massive invasion of leukocytes occurs in an allograft rejection, skin infection or in an injured area, and is also observed in various diseases including degenerative joint diseases, such as osteoarthritis, psoriasis, multiple sclerosis, asthma, rheumatoid arthritis, contact dermatitis and inflammatory bowel disease.

In such diseases, greater than 95% of myeloid cells move to and accumulate at the site of inflammation. Leukocytes are crucial agents of the inflammatory response, which exert antimicrobial, secretory and phagocytic activity. They gather in tissues where inflammation is occurring or needs to occur by producing a water-soluble mediator or through specific adhesion to various cells. In fact, anti-inflammatory agents such as nonsteroidal anti-inflammatory drugs (NSAIDs) or glucocorticoid exert therapeutic efficacy by preventing the adhesion and influx of leukocytes. In animal models, the inhibition of intercellular adhesion improves or prevents diseases or allograft rejection in animal models of autoimmune diseases. Recent clinical studies have revealed that humanized monoclonal antibodies inhibiting LFA-1/ICAM-1 or VLA-4/VCAM-1 interaction have significant efficacy and good safety on autoimmue diseases including psoriasis, multiple sclerosis and inflammatory bowel disease.

The uncontrolled invasion of leukocytes into endothelial cells, which is a key feature in the pathogenesis of inflammation-associated diseases, occurs by a multi-step process, which begins with leukocyte adhesion and binding to the surface of endothelial cells. The binding of leukocytes to endothelial cell surface is mediated by cell surface molecules present on the surface of leukocytes and endothelial cells [Bevilacqua, J. Clin. Invest. 11:767-804, 1993]. The cell surface molecules are overexpressed as a result of migration of leukocytes from the bloodstream.

The interaction between leukocytes and endothelial cells is a critical factor in many inflammatory diseases. For example, increased leukocyte-endothelial interaction leading to hepatic microperfusion disorders is proposed as a major contributor of hepatic failure [Croner et al., Microvasc. Res. 67:182-191, 2004]. For example, atherosclerosis is a typical inflammatory disease in which a number of inflammatory cells including T lymphocytes and activated macrophages are concentrated in the site of atherosclerosis. The accumulation and adhesion of monocytes in discrete segments of arterial endothelium is among the earliest detectable events in atherogenesis and is a central feature of the pathogenesis of atherosclerosis [Ross, Nature 362:801-809, 1993]. In this region, proinflammatory cytokines are abundant, which include interferon-gamma and tumor necrosis factor-alpha, regulating regional inflammatory response. A great number of adhesion molecules are expressed on the surface of monocytes [Valente et al., Circulation 86:11120-25, 1992], and endothelial cells overlying atherosclerotic lesions express a number of vascular ligands [Poston et al., Am. J. Pathol, 190:665-673, 1992].

The extravasation of leukocytes across the endothelial barrier is a critical event in the pathogenesis of inflammatory diseases such as rheumatoid arthritis. Endothelial cells participate in the basic mechanism of arthritis, by which various inflammation mediators, such as tumor necrosis factor-alpha and inflammation-inducing cytokines such as interleukin-1 beta, activate endothelial cells. This leads to elevated expression of endothelial cell adhesion molecules in rheumatoid arthritis, resulting in increased interaction between leukocytes and endothelial cells. The recruitment of leukocytes to vascular endothelial cells is also an important step of asthma.

In the airway of patients with asthma, there are increased numbers of activated eosinophils, CD25-positive T lymphocytes and immature macrophages with the phenotypic characteristics of blood monocytes. The expression of HLA class II increases in epithelial cells, macrophages, and other infiltrating cells [Arm et al., Adv. Immunol. 51:323-382, 1992].

An increased rate of leukocyte transmigration across the blood-brain barrier is a major symptom in multiple sclerosis. The interaction between tight junction proteins in leukocytes and those in endothelial cells contributes to the leukocyte extravasation to the central nervous system under physiological conditions, and the altered expression of tight junction proteins is a pathological prerequisite for multiple sclerosis [Worthylake et al., Curr. Opin. Cell Biol. 13:569-577, 2001].

As described above, since the adhesion of leukocytes to endothelial cells is important in a variety of diseases, the inhibition of intercellular adhesion using antibodies or peptide inhibitors is interesting as a new therapeutic strategy for diverse inflammatory and immune diseases.

With respect to the molecular biology, the following molecules participate in inflammation.

Cytokines: systemic inflammation, which is a general response to serious bacterial infections or traumatic injuries, may affect tissue systems distal to the early damage [Lush and Kvietys, Microcirculation 7:83-101, 2000]. Bacterial products and other inflammation-inducing mediators, released from affected tissues, induce the formation of inflammation-inducing mediators including tumor necrosis factor-alpha (TNF-alpha), interleukin-1 beta, gamma-interferon and interleukin-6. In sepsis, vascular endothelial damage promotes the production of TNF-alpha and interleukin-1 beta. These cytokines directly act on endothelial cells and enhance leukocyte adhesion [Pober et al., J. Immunol. 137:1893-1896, 1986; Dustin and Springer, J. Cell Biol. 107:321-331, 1988; Cotran and Pober, J. Am. Soc. Nephrol. 1:225-235, 1988]. These cytokines also activate blood neutrophils in blood and vascular endothelium [Arai et al., Annu Rev Biochem, 59:783-836, 1990]. For example, TNF-alpha induces a series of cytokines, chemokines and proteases by an autocrine or paracrine pathway [Ghezzi and Cerami, Methods Mol. Med. 98:1-8. 2004]. Interleukin-6 induces mononuclear-endothelial cell interaction and inflammatory damage through expression of adhesion molecules, thus initiating a process of atherosclerosis. Increased blood concentration of interleukin-6 involves vascular inflammation and development of atherosclerosis [Rader, N. Engl. J. Med. 343:1179-1182, 2000]. Interleukin-17 induces the expression of many mediators of inflammation, and is involved in the differentiation, maturation and chemotaxis of neutrophil[Witowski et al., Cell Mol Life Sci. 61:567-579, 2004]. Increased levels of interleukin-17 have been associated with several pathological conditions, including airway inflammation, rheumatoid arthritis, intraperitoneal abscesses and adhesions, inflammatory bowel disease, allograft rejection, psoriasis, cancer and multiple sclerosis.

Cell surface adhesion molecules: a plurality of inflammatory cytokines induce the expression of endothelial cell-lymphocyte adhesion molecules (ELAMs) on the cell surface [Nortamo et al., Eur. J. Immunol. 21:2629-2632, 1991]. They are divided into two classes: intercellular adhesion molecule-1 (ICAM-1) and endothelial cell-lymphocyte adhesion molecule-1 (ELAM-1) [Staunton et al., Cell 52:925-933, 1988]. In response to various mediators, vascular endothelium expresses specific cell surface glycoproteins. The binding and extravasation of blood leukocytes are achieved by interaction with a specific ligand or counter receptor [Bevilacqua et al., 1993, 1994]. Molecules participating in this process include intercellular adhesion molecule-1 (ICAM-1) as a ligand for CD18, selectins recognizing glycoonjugates on the leukocyte surface, and members of the immunoglobulin superfamily interacting with other members of the same family, leukocyte integrin molecules [Panes et al., J. Physiol. 269:H1955-1964, 1995; Khan et al., Microcirculation 10:351-358, 2003; Nelson et al., Blood 82:3253-3258, 1993; Bevilacqua and Nelson, J. Clin. Invest. 91:379-387, 1993]. Leukocyte rolling is regulated by selectins, and transmigration and adhesion of leukocytes on endothelial cells are triggered by the beta 2 integrin, Mac-1 (CD11b/CD18, aMb2, CR3), and LFA-1. Mac-1 and LFA-1 interact with a counter receptor expressed on the surface of endothelial cells, ICAM-1.

Current treatment approaches for inflammation are as follows.

According to a recent review [Ohmori et al., Nippon Yakuraigaku Zasshi 123:335-348, 2004], novel anti-allergic therapies have been developed. Various therapeutic agents are described, which include antibodies to T helper cell subtype 2 cytokine, decoy receptors, anti-receptor antibodies, anti-immunoglobulin E antibodies, anti-cell adhesion molecule antibodies, antisense oligonucleotides, keratinocyte modulators, inhibitors versus intracellular regulatory enzymes, and anti-histamines. Most of them are based on inhibiting various cellular constituents of allergic inflammation. At present, rheumatoid arthritis therapy involves a step-up approach in which doctors prescribe anti-inflammatory drugs, such as aspirin or ibuprofen, in an intermittent or periodical regime and later prescribe to patients having resistance to the primary prescription toxic DMARDs (disease-modifying anti-rheumatic drugs) affecting the body's immune system.

Ph. Nakashima exhibited that the progression of inflammatory abdominal aortic aneurysm (AAA) is inhibited by treatment of chimeric oligodeoxynucleotides against both NF-κB and Ets genes in a rat model. In a mouse model of lung inflammation caused by FasL, the injection of FasL into the lung results in a rapid increase in eosinophil invasion and proinflammatory agents. Also, the pretreatment of a FasL inhibitor, such as DcR3 analogue (decoy receptor 3 analogue), decreases the eosinophil invasion into the airway, resulting in remarkably decreased expression of granulocyte macrophage colony stimulating factor (GM-CSF) and macrophage inflammatory protein-2 (MIP-2) in bronchoalveolar lavage (BAL).

The prior arts associated with inflammation therapy are as follows.

The US5367056 patent describes the inhibition of the binding of polymorphonuclear leukocytes (PMNs) to endothelial cells by treatment of molecules or fragments thereof interrupting the binding to endothelial cell-leukocyte adhesion molecules (ELAMs) as receptors or ligands. This patent also describes antisense nucleotides and ribozymes for suppressing ELAM expression. This patent further describes a method for identifying molecules which inhibit the binding of ELAM to its ligand, and antibodies against ELAM and its ligands.

The US5863540 patent discloses a method of suppressing T cell activation by administrating a CD44 protein peptide or a derivative thereof in an amount sufficient to suppress T cell activation. Also disclosed is a method of inhibiting CD44-mediated cell adhesion or CD44-mediated monocyte IL1 release by administering to the CD44 protein peptide or derivative thereof in an amount sufficient to inhibit CD44-mediated cell adhesion or monocyte IL1 release. Further disclosed is a method of transporting a drug or cytotoxic agent to a site of inflammation by administering the CD44 protein peptide or derivative thereof linked to the drug or cytotoxic agent.

The US5912266 patent involves the inhibition of intercellular adhesion mediated by the beta 2 integrin family of cell surface molecules. Through this inhibitory action, a pharmaceutical composition according to this patent is useful for inhibiting or treating inflammatory and other pathological responses associated with cell adhesion. This patent also discloses a method of inhibiting or treating pathological conditions where leukocytes and lymphocytes cause cellular or tissue damage.

The WO03026692 patent relates to the therapeutic use of an antibody against CD3 antigen complexes in patients with chronic articular inflammation and rheumatoid arthritis.

The EP1304379 patent relates to a humanized anti-CD18 antibody comprising a portion or the whole of an antigen-determining region capable of binding to CD18 antigen.

The US6689869 patent describes the use of a humanized anti-CD18 antibody in inhibiting influx of leukocytes into the lung and other organs during sepsis, and other infectious or non-infectious traumas. The humanized anti-CD18 antibody can be used for inhibiting the ingress of leukocytes into the lung and other organs in patients having endotoxic shock or adult respiratory distress syndrome. The antibody can administered to treat asthma or leukocyte-mediated reperfusion damage post thrombolytic therapy. Also, the antibody can be used to reduce or eliminate inflammation in a patient being administered with an anti-infective agent, or to assist in the administration of a therapeutic drug to a patient during anticancer chemotherapy.

The US5821336 patent describes polypeptides having a molecular weight of 160 kD, which are mediators or precursors for mediators of inflammation, derivatives thereof, such as mutants and fragments, and processes for their preparation. Nucleotide sequences coding for the polypeptides and derivatives, vectors comprising the nucleotide sequences, antibodies against the polypeptides or their derivatives and antibody derivatives are also included in the scope of this patent. Moreover described are diagnostic and therapeutic methods for inflammatory conditions and Hodgkin's lymphomas using the antibodies and antibody derivatives.

EP 1 059 533 A1 discloses an immunochemical assay for an anti-HM1.24 antibody. No association of HM 1.24 with any disease is disclosed therein.

EP 1 394 274 A2 discloses a method of testing for bronchial asthma or chronic obstructive pulmonary disease, which is based on the identification of numerous marker genes whose expression levels varied between respiratory epithelial cells that had been stimulated with IL-13 to induce the goblet cell differentiation, and unstimulated respiratory epithelial cells.

EP 0 972 524 A1 discloses the inhibition of lymphocyte activation by an anti-HM 1.24 antibody. It is proposed to use the antibody in the treatment of autoimmune diseases, organ transplant rejection or allergy.

### Disclosure of the Invention

Inflammation requires at least three sequential steps to attracting immune cells comprising leukocytes into the site of inflammation, as follows: (1) immune cells including leukocytes are aggregated through intercellular adhesion; (2) the aggregated immune cells migrate and are recruited to the site of inflammation, where they transduce related signals into endothelial cells through adhesion to endothelial cells; (3) T lymphocytes are activated and secrete cytokines, such as interleukin-2, to amplify the inflammatory response.

The present inventors found that Bst2 or Damp1 protein mediates homotypic adhesion of immune cells or heterotypic adhesion between immune cells and endothelial cells, which play crucial roles in inflammation, and further found that an antagonist of the protein acts in the major three steps of inflammation and can thus be used in the treatment of inflammation-associated diseases, thereby leading to the present invention.

Therefore, the present description relates to the Bst2 protein or a fragment thereof, which has an inflammation-suppressing effect by inhibiting intercellular adhesion.

In particular, the present invention provides a pharmaceutical composition for use in preventing or treating an inflammatory disease, comprising as an active ingredient (i) a fragment of the Bst2 protein comprising the amino acid sequence of SEQ ID NO: 2 having an inflammation-suppressing effect, wherein the fragment of the Bst2 protein has a deletion of the whole or a portion of an intracellular domain thereof and comprises the amino acid sequence of SEQ ID NO: 5; or (ii) a variant of the protein of (i) characterized by having an amino acid sequence different from the sequence of the protein of (i) in one or more amino acid residues by a deletion, an insertion, a non-conservative or conservative substitution or a combination thereof, wherein the variant has an inflammation-suppressing effect, wherein the inflammatory disease is asthma. The invention is defined by the claims.

### Brief Description of the Drawings

The above and other objects, features and other advantages of the present invention will be more clearly understood from the following detailed description taken in conjunction with the accompanying drawings, in which:
Fig. 1 is an amino acid sequence alignment showing sequence similarity between human Bst2 and mouse Damp1;
Fig. 2 shows the locations of PCR primers used in a process for cloning a human Bst2 soluble fragment and a mouse Damp1 soluble fragment into an expression vector;
Fig. 3 shows the results of electrophoresis analysis of a human Bst2 soluble fragment and a mouse Damp1 soluble fragment;
Fig. 4 shows the expression pattern of Bst2 gene during homotypic aggregation of U937 cells;
Fig. 5 shows the promoting effect of Bst2 overexpression on homotypic aggregation of U937 cells;
Fig. 6 shows the effect of a Bst2 soluble fragment on homotypic aggregation of U937 cells;
Fig. 7 shows the effect of a Bst2 soluble fragment on intercellular adhesion between human vascular endothelial (HUVEC) cells and U937 cells;
Fig. 8 shows the dose-dependent effect of a Bst2 soluble fragment on intercellular adhesion between HUVEC cells and U937 cells;
Fig. 9 shows the effect of Bst2 siRNA on intercellular adhesion between HUVEC cells and U937 cells;
Fig. 10 shows the effect of Bst2 siRNA on intercellular adhesion between HUVEC cells and U937 cells upon Bst2 overexpression;
Fig. 11 shows the effect of Bst2 overexpression on aggregation of Jurkat cells and interleukin-2 (IL-2) production in Jurkat cells;
Fig. 12 shows the effect of a Bst2 soluble fragment and Bst2 siRNA on aggregation of Jurkat cells;
Fig. 13 is a graph showing the effect of a Bst2 soluble fragment on aggregation of Jurkat cells and IL-2 production;
Fig. 14 shows the change in the number of sedimented immune cells upon treatment of a Bst2 soluble fragment;
Fig. 15 shows the decreased levels of cytokines upon treatment of a Bst2 soluble fragment;
Fig. 16 shows the functional similarity between human Bst2 and mouse Damp1;
Fig. 17 shows the inhibitory effect of a mouse Damp1 soluble fragment on asthma induced in mice;
Fig. 18 shows PEG moieties used in preparation of PEG-conjugated forms of a Bst2 soluble fragment;
Fig. 19 shows the improved metabolic degradation of PEG-conjugated Bst2; and
Fig. 20 shows the expression and distribution of Bst2 in inflammation-associated diseases.

### Best Mode for Carrying Out the Invention

The present present description relates to antagonists of Bst2 (Bone marrow Stromal Antigen-2) protein, which participate in intercellular adhesion during inflammation.

The present inventors, through studies using (1) a homotypic aggregation model of human U937 monocytic cells to investigate the effect of Bst2 on aggregation of immune cells, (2) a heterotypic aggregation model between U937 cells and HUVEC cells to investigate the effect of Bst2 on intercellular adhesion between immune cells and endothelial cells, (3) a Jurkat T-cell model to investigate the effect of Bst2 on T lymphocyte activation, found that Bst2 protein participates in an inflammation process in which lymphocytes migrate to the site of inflammation, recognize extracellular matrix components to interact with cells, and adhere to the cells. The present inventors further found that an antagonist of Bst2 protein effectively inhibits such intercellular adhesion and is thus able to effectively treat inflammatory diseases.

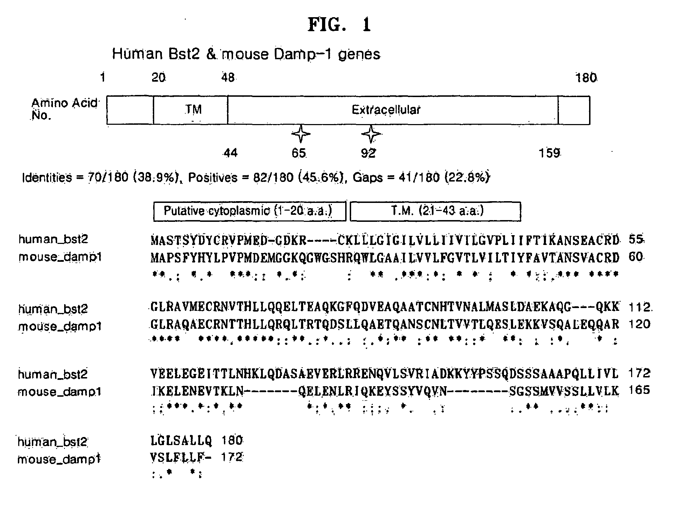

The Bst2 protein was initially identified in bone marrow stromal cells and is considered to be involved in the differentiation and proliferation of cells. A cDNA encoding Bst2 was cloned in 1995, and the BST-2 gene was found to be located on human chromosome 19p13.2 [Ishikawa et al., Genomics 26:527-534, 1995]. The Bst2 gene consists of five exons and four introns. Bst2 is a 30- to 36-kD type II transmembrane protein consisting of 180 amino acids [Ohtomo et al., Biochem. Biophys. Res. Commun. 258:583-591, 1999]. Damp1 gene, a mouse homologue of human Bst2 gene, has 45% DNA sequence identity to the human Bst2 gene, and as shown in Fig. 1, has less than 40% amino acid sequence similarity to human Bst2. The Bst2 protein is predominantly expressed in the liver, lung, heart and placenta, and in lower levels in the pancreas, kidneys, skeletal muscle and brain. BST-2 surface expression on fibroblast cells accelerates the stromal cell-dependent growth of murine bone marrow-derived pre-B cells. This result suggests that Bst2 regulates pre-B-cell growth or plays a critical role in B cell activation in rheumatoid arthritis. Bst2 is also overexpressed in some types of cancer, including oral cancer, breast cancer, adenoma and cervical cancer.

With respect to Bst2 protein, the isolation and expression of a gene encoding Bst2 protein (EP1033401), and the use of the Bst2 protein in cancer diagnosis (WO01/57207 and WO01/51513) have been reported.

The term "Bst2 or Damp1 antagonist", as used herein, refers to a substance that inhibits, blocks or reduces the activity of Bst2 or Damp1 protein acting on a mechanism inducing inflammation. The action mechanism of the antagonist is not specifically limited. Examples of the antagonist include substances affecting gene expression of Bst2 or Damp1, such as transcription and translation, and substances converting an active protein to an inactive form. Such antagonists include single compounds, such as organic or inorganic compounds; polymeric compounds, such as proteins, nucleic acids, carbohydrates and lipids; and composites of multiple compounds.

In a detailed aspect, the antagonist includes Bst2 protein or a fragment thereof, which has an inflammation-suppressing effect by inhibiting intercellular adhesion. In another detailed aspect, the antagonist includes Damp1 protein or a fragment thereof, which has an inflammation-suppressing effect by inhibiting intercellular adhesion.

The Bst2 or Damp1 protein is divided into three domains: cytoplasmic, transmembrane and extracellular domains, and an intracellular domain contains cytoplasmic and transmembrane domains. A "Bst2 protein fragment" or "Damp1 protein fragment" is not specifically limited so long as it has an inflammation-suppressing effect by inhibiting intercellular adhesion, but is preferably a Bst2 or Damp1 protein having a deletion of the whole or a portion of the intracellular domain. In a detailed embodiment, the Bst2 protein fragment is a Bst2 protein fragment comprising the amino acid sequence of SEQ ID No. 5. In another detailed embodiment, the Damp1 protein fragment is a Damp1 protein fragment comprising the amino acid sequence of SEQ ID No. 6. The Bst2 protein fragment and Damp1 protein fragment were found to effectively inhibit the intercellular adhesion induced by Bst2 or Damp1. In the specification of the present invention, the terms "soluble fragment", "soluble protein", "soluble protein fragment" and "decoy protein" are interchangeably used.

The present disclosure includes a protein having a native amino acid sequence of the Bst2 protein, Damp1 protein or a fragment thereof, having an inflammation-suppressing effect by inhibiting intercellular adhesion, and an amino acid sequence variant of the native protein. The "variant" means a protein or a fragment thereof, which has a sequence different from a native amino acid sequence of Bst2 protein, Damp1 protein or a fragment thereof in one or more amino acid residues, by a deletion, an insertion, a non-conservative or conservative substitution or a combination thereof. For examples, amino acid exchanges in proteins and peptides which do not generally alter the activity of the proteins or peptides are known in the art (H. Neurath, R. L. Hill, The Proteins, Academic Press, New York, 1979). The most commonly occurring exchanges are Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu and Asp/Gly, in both directions.

In addition, the protein or fragment thereof may be in the form of having native sugar chains, increased sugar chains compared to a native form or decreased sugar chains compared to the native form, or may be in a deglycosylated form. The increase, decrease or removal of sugar chains of the protein may be achieved by an ordinary method, such as a chemical method, an enzymatic method, or a genetic engineering method using a microorganism.

The protein or fragment, if desired, may be modified by phosphorylation, sulfation, acrylation, methylation, farnesylation, and the like.

Such variants include a functional equivalent exerting activity identical to the native form or a protein having a modification enhancing or reducing physical and chemical properties. Preferred is a variant having a modified physicochemical property. For example, the variant has enhanced structural stability against external environments including physical factors, such as temperature, humidity, pH, electrolytes, reducing sugars, pressure, dryness, freezing, interfacial tension, light, repeated freezing and thawing, high concentrations, and the like; and chemical factors, such as acids, alkalis, neutral salts, organic solvents, metal ions, oxidizing and reducing agents, proteases, and the like.

The Bst2 or Damp1 protein, a fragment thereof, or a variant thereof, which has an inflammation-suppressing effect by inhibiting intercellular adhesion, may be naturally isolated or synthesized (Merrifleld, J. Amer. Chem. Soc., 85:2149-2156, 1963), or may be prepared by a recombination method based on DNA sequence (Sambrook et. al., Molecular Cloning, Cold Spring Harbour Laboratory Press, New York, USA, 2nd Ed., 1989). When a genetic recombination technique is used, a desired protein, may be obtained by inserting a nucleic acid encoding the Bst2 protein, Damp1 protein, a fragment thereof or a variant thereof into a suitable expression vector, transforming a host cell with the expression vector, culturing the host cell to express the desired protein, and recovering the produced protein from the culture.

The Bst2 protein, Damp1 protein, or a fragment thereof, which has an inflammation-suppressing effect by inhibiting intercellular adhesion, may be in a monomeric or multimeric form. A multimer may be formed by various methods commonly known in the art, and the method for forming a multimer is not specifically limited. For example, a multimer may be prepared using a sequence inducing multimer formation, for example, isoleucine zipper (ILZ) sequence inducing trimer formation, or surfactant protein-D (SP-D) inducing dodecamer formation. Otherwise, a multimer may be prepared by conjugating two or more polypeptides, which each have been produced in a monomeric form, for example, using a linker.

In another aspect, disclosed herein is a nucleic acid molecule encoding Bst2 protein or a fragment thereof, which has an inflammation-suppressing effect by inhibiting intercellular adhesion. Also, there is disclosed a nucleic acid molecule encoding Damp1 protein or a fragment thereof, which has an inflammation-suppressing effect by inhibiting intercellular adhesion.

The nucleic acid encoding Bst2 protein, Damp1 protein, or a fragment thereof, which has an inflammation-suppressing effect by inhibiting intercellular adhesion, may be modified in one or more bases, by a substitution, a deletion, an insertion, or a combination thereof, as long as it encodes a protein or fragment having activity identical to the native form. Such nucleic acid molecules may be single-stranded or double-stranded, and may be DNA molecules or RNA (mRNA) molecules.

In a further aspect, disclosed herein is a vector comprising a nucleic acid molecule encoding Bst2 protein or a fragment thereof, which has an inflammation-suppressing effect by inhibiting intercellular adhesion.

The term "vector", as used herein, which describes a vector capable of expressing a protein of interest in a suitable host cell, refers to a genetic construct that comprises essential regulatory elements to which a gene insert is operably linked in such a manner as to be expressed in a host cell.

The term "operably linked", as used herein, refers to a functional linkage between a nucleic acid expression control sequence and a second nucleic acid sequence coding for a target protein in such a manner as to allow general functions. For example, a promoter may be operably linked to a nucleic acid sequence coding for a protein and affect the expression of the coding sequence. The operable linkage to a vector may be prepared using a genetic recombinant technique well known in the art, and site-specific DNA cleavage and ligation may be achieved using enzymes generally known in the art.

The vector includes, but is not limited to, plasmid vectors, cosmid vectors, bacteriophage vectors and viral vectors. A suitable expression vector includes expression regulatory elements, such as a promoter, an initiation codon, a stop codon, a polyadenylation signal and an enhancer, as well as signal sequences for membrane targeting or secretion, and may vary according to intended use. An expression vector may also include a selectable marker that allows selection of host cells containing the vector. A replicable expression vector may include a replication origin. The signal sequence includes, but is not limited to, PhoA signal sequence and Omp signal sequence for *Escherichia* species as hosts; α-amylase signal sequence and subtilisin signal sequence for *Bacillus* species as hosts; MF α signal sequence and SUC2 signal sequence for yeast host cells; and insulin signal sequence, α-interferon signal sequence, antibody molecule signal sequence and tPA (tissue plasminogen activator) signal sequence for animal host cells.

In yet another aspect, disclosed herein is a transformant transformed with the vector.

The transformation includes any method by which nucleic acids can be introduced into organisms, cells, tissues or organs, and, as known in the art, may be performed by selecting suitable standard techniques according to host cells. These methods include, but are not limited to, electroporation, protoplast fusion, calcium phosphate (CaPO₄) precipitation, calcium chloride (CaCl₂) precipitation, agitation with silicon carbide fiber, agrobacterium-mediated transformation, and PEG-, dextran sulfate- and lipofectarnine-mediated transformation.

Protein expression systems in host cells are well known in the art. Host cells most suitable for objects may be selected and used because expression levels, modification, or the like of proteins vary depending on host cells. Host cells include, but are not limited to, prokaryotic cells such as *Escherichia coli, Bacillus subtilis, Streptomyces, Pseudomonas, Proteus mirabilis* or *Staphylococcus.* Also, eukaryotic cells useful as host cells include lower eukaryotic cells, such as fungi (e.g., *Aspergillus*) and yeasts (e.g., *Pichia pastoris, Saccharomyces cerevisiae, Schizosaccharomyces, Neurospora crassa*), and cells derived from higher eukaryotes, such as insect cells, plant cells and mammalian cells. Genetically manipulated cells may be also used as host cells. Examples of such cells include a strain harboring a genetically-modified modification pathway for sugar chains by manipulating an enzyme involving protein processing to provide a humanized sugar chain.

In still another aspect, disclosed herein is a method of preparing Bst2 protein, Damp1 protein, or a fragment thereof, which has an inflammation-suppressing effect by inhibiting intercellular adhesion, comprising culturing the transformant.

The cultivation of host cells may be performed under culture conditions suitable for expressing Bst2 protein, Damp1 protein or fragments thereof through a method generally known to those skilled in the art.

Proteins or fragments thereof expressed in host cells, namely, Bst2 protein, Damp1 protein or fragments thereof, may be purified by ordinary methods, which may be used separately or in combination, for example, salting out (e.g., ammonium sulfate precipitation, sodium phosphate precipitation, etc.), solvent precipitation (e.g., protein fraction precipitation using acetone, ethanol, etc.), dialysis, chromatographic methods such as gel filtration chromatography, ion exchange chromatography and reverse phase chromatography, and ultrafiltration.

The Bst2 protein, Damp1 protein or fragment thereof, which has an inflammation-suppressing effect by inhibiting intercellular adhesion, may be modified by a non-peptide polymer.

In a further detailed aspect, the antagonist includes non-peptide polymer-modified Bst2 protein or a fragment thereof, which has an inflammation-suppressing effect by inhibiting intercellular adhesion.

In yet another detailed aspect, the antagonist includes non-peptide polymer-modified Damp1 protein or a fragment thereof, which has an inflammation-suppressing effect by inhibiting intercellular adhesion.

The term "modification", as used herein, indicates a process in which a non-peptide polymer is linked to Bst2 protein, Damp1 protein, or a fragment thereof.

The term "non-peptide polymer", as used herein, refers to a biocompatible polymer in which two or more repeating units are linked to each other. Examples of the non-peptide polymer include polyethylene glycol, polypropylene glycol (PPG), co-poly(ethylene/propylene) glycol, polyoxyethylene (POE), polyurethane, polyphosphazene, polysaccharide, dextran, polyvinyl alcohol, polyvinyl pyrrolidone, polyvinyl ethyl ether, polyacryl amide, polyacrylate, polycyanoacrylate, lipid polymer, chitins, hyaluronic acid, and heparin. A preferred non-peptide polymer is polyethylene glycol.

The linkage of the Bst2 protein, Damp1 protein or fragments thereof with a non-peptide polymer include covalent bonds and all types of non-covalent bonds, such as hydrogen bonds, ionic interactions, van der Waals forces and hydrophobic interactions. Preferably, the polymer is linked with a protein through a specific reactive group. Examples of reactive groups of the polymer include an aldehyde group, a propionic aldehyde group, a butyl aldehyde group, a maleimide group, a ketone group, a vinyl sulfone group, a thiol group, a hydrazide group, a carbonyldimidazole (CDI) group, a nitrophenyl carbonate (NPC) group, a trysylate group, an isocyanate group, and succinimide derivatives. The non-peptide polymer reacts with reactive groups of a polypeptide, for example, an N-terminus, a C-terminus or/and side chain of amino acid residues (e.g., side chain of a lysine residue, a histidine residue or a cysteine residue).

The Bst2 protein, Damp1 protein, or fragment thereof, which has an inflammation-suppressing effect by inhibiting intercellular adhesion, may be linked with a non-peptide polymer in a molar ratio of 1:1 to 1:10, preferably 1:1 to 1:2. When the Bst2 protein, Damp1 protein, or fragment thereof, is modified by two or more non-peptide polymers, the non-peptide polymers are identical or different.

The proteins may have improved *in vivo* stability and metabolism through modification with non-peptide polymers.

In still another detailed aspect, the antagonist includes a siRNA molecule comprising an antisense RNA strand complementary to Bst2 mRNA or Damp1 mRNA and a sense RNA strand complementary to the antisense RNA strand and inducing Bst2- or Damp1-specific RNA interference, the siRNA molecule having an inflammation-suppressing effect by inhibiting intercellular adhesion.

The term "siRNA", as used herein, refers to a short double-stranded RNA molecule that is able to induce RNA interference (RNAi) through cleavage of the target mRNA. The term "specific" or "specific to", as used herein, means an ability to suppress only a target gene while not affecting other genes in cells. Described herein are siRNA molecules specific to Bst2 or Damp1.

The siRNA is a nucleic acid molecule that is preferably 10 to 40, more preferably 20 to 30, even more preferably 19 to 25, base pairs long.

In a detailed aspect, Bst2-specific siRNA molecules were prepared, which each containing an antisense RNA strand complementary to mRNA expressed from a sequence having any one of the nucleotide sequences of SEQ ID Nos. 18 to 40, and a sense RNA strand complementary to the antisense strand.

The siRNA is not limited to contain matches in which both strands are perfectly paired, but may also contain unpaired regions, such as mismatches (corresponding bases are not complementary) or bulges (corresponding bases are not present in the other strand). Typically, the siRNA is preferable to have 90% or higher homology to Bst2 mRNA or Damp1 mRNA.

The terminal structure of siRNA may be either blunt or cohesive. The cohesive end structure may be either 5' overhang or 3' overhang. The number of overhanging nucleotides is not limited. For example, the overhang consists of 1 to 8 nucleotides, preferably 2 to 6 nucleotides. Also, as long as siRNA is able to retain its gene silencing effect on the target gene, for example, in the overhanging portion at its one end, siRNA may contain a low molecular weight RNA molecule (e.g., a natural RNA molecule such as tRNA, rRNA or viral RNA, or an artificial RNA molecule). It is not necessary for the terminal structure of siRNA to have the cut-off structure at both ends, and it may have a stem-loop structure in which ends of one side of double-stranded RNA are connected by a linker RNA. The length of the linker is not specifically limited as long as it does not disrupt the pairing of the stem region.

The siRNA may be prepared by a method in which siRNA is directly synthesized *in vitro* and introduced into cells by transfection, or another method in which a siRNA expression vector or PCR-induced siRNA expression cassette, which is constructed to express siRNA in cells, is transformed or transfected into cells. The determination of a method for preparing siRNA and introducing siRNA into cells or animals may vary according to test purposes and cellular biological functions of target gene products.

The siRNA may be mixed with an agent stimulating its influx, for example, liposomes (U.S. Pat. Nos. 4,897,355, 4,394,448, 4,235,871, 4,231,877, 4,224,179, 4,753,788, 4,673,567, 4,247,411 and 4,814,270) or a lipophilic carrier selected from multiple sterols including cholesterol, cholate and deoxycholate.

In yet another detailed aspect, the antagonist includes antisense nucleic acid molecules specific to Bst2 mRNA or Damp1 mRNA.

The term "antisense nucleic acids", as used herein, refers to DNA, RNA or derivatives thereof containing a nucleic acid sequence complementary to a specific mRNA sequence, and bind to a complementary sequence in mRNA and inhibit translation of mRNA into a protein. The antisense sequences mean DNA or RNA sequences that are complementary to Bst2 mRNA or Damp1 mRNA and are able to bind Bst2 mRNA or Damp1 mRNA, and may inhibit translation, cytoplasmic translocation or maturation of Bst2 mRNA or Damp1 mRNA or all other activities essential for overall biological functions.

The antisense nucleic acids may be modified at one or more positions of bases, sugars or backbones in order to have improved effectiveness (De Mesmaeker et al., Curr Opin Struct Biol., 5(3):393-55, 1995). The nucleic acid backbone may be modified, for example, with phosphorothioates, phosphotriesters, methyl phosphonates, short chain alkyl or cycloalkyl intersugar linkages, or short chain heteroatomic or heterocyclic intersugar linkages. Also, the antisense nucleic acids may contain one or more substituted sugar moieties. The antisense nucleic acids may also contain modified bases. Examples of the modified bases include hypoxanthine, 6-methyladenine, 5-methyl-pyrimidines (especially 5-methylcytosine), 5-hydroxymethylcytosine (HMC), glycosyl HMC, gentiobiosyl HMC, 2-aminoadenine, 2-thiouracil, 2-thiothymine, 5-bromouracil, 5-hyroxymethyluracil, 8-azaguanine, 7-deazaguanine, N₆(6-aminohexyl)adenine, and 2,6-diaminopurine. In addition, the antisense nucleic acids may be chemically bonded to one ore more moieties or conjugates enhancing the activity and cellular uptake of the antisense nucleic acids. For example, liphophilic moieties include, but are not limited to, a cholesterol moiety, a cholesteryl moiety, cholic acid, a thioether, a thiocholesterol, an aliphatic chain, a phospholipid, a polyamine chain, a polyethylene glycol chain, adamantane acetic acid, a palmityl moiety, an octadecylamine moiety and a hexylamino-carbonyl-oxycholesterol moiety. A method of preparing oligonucleotides comprising lipid moieties is well known in the art (U.S. Pat. Nos. 5,138,045, 5,218,105 and 5,459,255). The modified nucleic acids may have enhanced stability in the presence of nucleases and enhanced binding affinity to target mRNA.

Antisense RNA may be synthesized *in vitro* by an ordinary method and administered to the body, or may be synthesized *in vivo*. A method for synthesizing antisense RNA *in vitro* employs RNA polymerase I. A method for synthesizing antisense RNA *in vivo* involves performing transcription of antisense RNA using a vector containing a multicloning site (MCS) in the opposite direction. Such antisense RNA preferably contains a translation stop codon in its sequence to block translation into a peptide sequence.

Described herein is a composition for preventing or treating inflammatory diseases, comprising one or more selected from among, as described above, Bst2 protein or a fragment thereof having an inflammation-suppressing effect by inhibiting intercellular adhesion; Damp1 protein or a fragment thereof having an inflammation-suppressing effect by inhibiting intercellular adhesion; non-peptide polymer-modified Bst2 protein or a fragment thereof having an inflammation-suppressing effect by inhibiting intercellular adhesion; non-peptide polymer-modified Damp1 protein or a fragment thereof having an inflammation-suppressing effect by inhibiting intercellular adhesion; siRNA nucleic acid molecules specific to Bst2 or Damp1; and antisense nucleic acid molecules specific to Bst2 or Damp1.

The term "prevention", as used herein, means all activities that inhibit inflammatory diseases or delay incidence of inflammatory diseases through administration of the composition. The term "treatment", as used herein, refers to all activities that alleviate and beneficially affect inflammatory diseases.

The term "inflammatory diseases", as used herein, refers to all diseases that result from the body's defense responses or infectious responses against harmful influences in states (physical, chemical and biological states) of having symptoms such as redness, swelling, tenderness, pain, fever and dysfunction. The present composition may be used for preventing or treating all types of inflammatory diseases induced by Bst2 overexpression. In fact, Bst2 has been identified to be overexpressed in various inflammatory diseases including asthma, atherosclerosis, rheumatoid arthritis, psoriasis, Crohn's disease and ulcerative colitis. Thus, diseases which may be prevented or treated by the present composition include atherosclerosis, rheumatoid arthritis, asthma, sepsis, ulcerative colitis, multiple sclerosis, acute myocardial infarction, heart attack, psoriasis, contact dermatitis, osteoarthritis, rhinitis, Crohn's disease and autoimmune diseases.

The present composition may be applied to humans, as well as to livestock whose inflammatory diseases can be inhibited or reduced by administration of Bst2 or Damp1, such as bovine, horses, sheep, swine, goats, camels, antelopes, dogs and cats. In this context, the present inventors found that human Bst2 and mouse Damp1 have functional similarity and act on cells having the same origin as well as a different origin.

The present composition may be administered in a pharmaceutically effective amount. The term "pharmaceutically effective amount", as used herein, refers to an amount sufficient for treatment of diseases, which is commensurate with a reasonable benefit/risk ratio applicable for medical treatment. An effective dosage amount of the composition may be determined depending on the type of disease, severity of the illness, the patient's age and gender, drug activity, drug sensitivity, administration time, administration routes, excretion rates of a drug, duration of treatment, drugs used in combination with the composition; and other factors known in medical fields. The present composition may be administered as individual therapeutic agents or in combination with other therapeutic agents, and may be administered sequentially or simultaneously with conventional therapeutic agents. This administration may be single or multiple dosing. Taking all factors into consideration, it is important to conduct administration with a minimum of doses capable of giving the greatest effects with no adverse effects, and the doses may be readily determined by those skilled in the art.

The present composition may be administered along with a pharmaceutically acceptable carrier. For oral administration, the pharmaceutically acceptable carrier may include binders, lubricants, disintegrators, excipients, solubilizers, dispersing agents, stabilizers, suspending agents, coloring agents and perfumes. For injectable preparations, the pharmaceutically acceptable carrier may include buffering agents, preservers, analgesics, solubilizers, isotonic agents and stabilizers. For topical administration, the pharmaceutically acceptable carrier may include bases, excipients, lubricants and preservers. The composition of the present invention may be formulated into a variety of dosage forms in combination with the aforementioned pharmaceutically acceptable carriers. For example, for oral administration, the composition may be formulated into tablets, troches, capsules, elixirs, suspensions, syrups or wafers. For injectable preparations, the composition may be formulated into a unit dosage form, such as a multidose container or an ampule as a single-dose dosage form.

The present composition may be administered orally, or parenterally, i.e., by intravenous, subcutaneous, intranasal or intraperitoneal administration, to humans or animals. The parenteral administration includes injection methods such as subcutaneous, intramuscular or intravenous injection, and drip injection. In addition, the present composition may be formulated into a variety of administration modes according to ordinary methods.

Disclosed herein is a method of suppressing an inflammatory response, comprising administering a substance inhibiting intercellular adhesion mediated by Bst2 or Damp1 protein.

The term "substance inhibiting intercellular adhesion mediated by Bst2 or Damp1 protein", as used herein, indicates an antagonist of Bst2 or Damp1 protein, and includes single compounds, such as organic or inorganic compounds; polymeric compounds, such as proteins, nucleic acids, carbohydrates and lipids, and composites of multiple compounds. In detail, the substance includes, as described above, Bst2 protein or a fragment thereof having an inflammation-suppressing effect by inhibiting intercellular adhesion; Damp1 protein or a fragment thereof having an inflammation-suppressing effect by inhibiting intercellular adhesion; non-peptide polymer-modified Bst2 protein or a fragment thereof having an inflammation-suppressing effect by inhibiting intercellular adhesion; non-peptide polymer-modified Damp1 protein or a fragment thereof having an inflammation-suppressing effect by inhibiting intercellular adhesion; siRNA nucleic acid molecules specific to Bst2 or Damp1; and antisense nucleic acid molecules specific to Bst2 or Damp1.

The aforementioned inflammatory diseases may be prevented or treated by inhibiting inflammatory responses.

In still another detailed aspect, the present disclosure relates to preventing or treating inflammatory diseases, comprising administering to a patient one or more proteins selected from among Bst2 protein or a fragment thereof having an inflammation-suppressing effect by inhibiting intercellular adhesion; Damp1 protein or a fragment thereof having an inflammation-suppressing effect by inhibiting intercellular adhesion; non-peptide polymer-modified Bst2 protein or a fragment thereof having an inflammation-suppressing effect by inhibiting intercellular adhesion; and non-peptide polymer-modified Damp1 protein or a fragment thereof having an inflammation-suppressing effect by inhibiting intercellular adhesion.

In still another detailed aspect, the present disclosure relates to preventing or treating inflammatory diseases, comprising administering to a patient one or more nucleotides selected from among a siRNA molecule comprising an antisense RNA strand complementary to Bst2 mRNA or Damp1 mRNA and a sense RNA strand complementary to the antisense RNA strand and inducing Bst2- or Damp1-specific RNA interference; and an antisense nucleic acid molecule specific to Bst2 mRNA or Damp1 mRNA.

A better understanding of the present invention may be obtained through the following examples which are set forth to illustrate, but are not to be construed as the limit of the present invention.

### EXAMPLE 1: Cell culture

A human monocytic cell line U937 (ATCC, U.S; Cat. CRL-1593.2) was suspension-cultured in RPMI-1640 (Gibco-BRL) supplemented with 10% fetal bovine serum (FBS; Gibco-BRL), 100 U/ml of penicillin (Gibco-BRL) and 100 µg/ml of streptomycin (Gibo-BRL) at 37°C under a 5% CO₂ atmosphere.

Human umbilical vein endothelium cell line HUVEC (Cambrex, U.S.; Cat. CC-2517A) was subcultured in EGM-2 medium (Cambrex, U.S.) supplemented with 10% FBS at 37°C under a 5% CO₂ atmosphere. In the following examples, cells were pretreated with 0.5% FBS, instead of 10% FBS, for 16 hrs. According to given conditions, cells were pretreated with human recombinant interferon-gamma (10 ng/ml, Cambiochem, U.S.) and PMA (1 ng/ml, Cambiochem) or a medium for a predetermined period of time.

A mouse monocytic cell line WEHI-274.1 (ATCC, Cat. CRL-1679), and a mouse endothelial cell line, SVEC 4-10 (ATCC, Cat. CRL-2181), were cultured and pretreated according to the same method as in the human cell lines.

A human T-lymphocyte cell line Jurkat (ATCC, TIB152 clone) was suspension-cultured in RPMI-1640 (Gibco-BRL) supplemented with 10% FBS, 100 U/ml of penicillin and 100 µg/ml of streptomycin at 37°C under a 5% CO₂ atmosphere.

Protein expression and purification were carried out using CHO-S cells (Invitrogen, Cat. 11619-012). CHO-S cells were suspension-cultured in F12/HAM (Gibco-BRL) medium supplemented with 10% FBS, 100 U/ml of penicillin and 100 µg/ml of streptomycin at 37°C under 5% CO₂ atmosphere.

### EXAMPLE 2: Cloning of human Bst2 gene and mouse Damp1 gene

An expression vector of histidine-tagged Bst2 was constructed as follows. Full-length cDNA (NM004335; SEQ ID No. 1) of human Bst2 gene was synthesized by Origene Technologies (USA), and amplified by PCR using Pfu ultra HF DNA polymerase (Stratagene) in a volume of 50 µl. A PCR product was cloned into a pCMV HA vector (Clontech) using SalI and NotI.

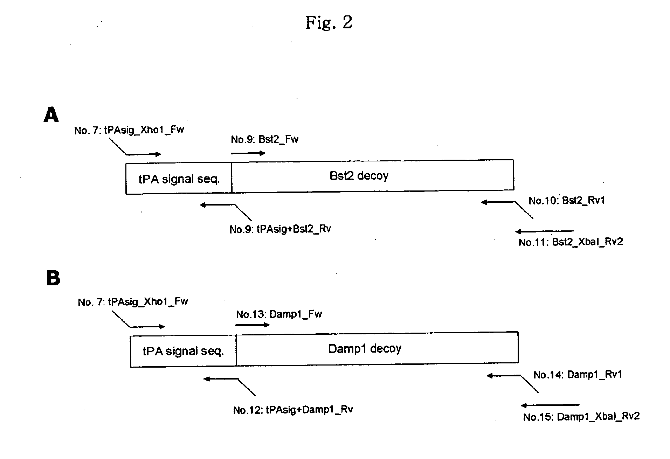

Vectors for expressing soluble fragments of Bst2 and Damp1 were constructed as follows. Fig. 2 shows the locations of PCR primers (SEQ ID Nos. 7 to 15) used in cloning the soluble fragments. A DNA fragment coding for the extracellular region of human Bst2 protein was obtained by PCR, and was fused at the N-terminus to a signal sequence P of tPA (tissue Plasminogen activator) to promote extracellular secretion after being expressed. The DNA fragment was also fused at the C-terminus to a six-histidine tag to facilitate determination of protein expression levels and protein purification. The Bst2 soluble fragment did not contain 11 amino acid residues at the C-terminus and also did not contain the transmembrane and cytoplasmic domains. The PCR product was treated with a final concentration of 0.8% dimethyl sulfoxide (DMSO; Sigma), digested with BamHI and XbaI, and cloned into a pCDNA 3.1 vector (Invitrogen).

Full-length cDNA (NM 198095; SEQ ID No. 3) of mouse Damp1 gene was obtained by RT-PCR using mRNA isolated from mouse liver. A RT-PCR product was digested with BamHI and XbaI and cloned into pCDNA 3.1 (Invitrogen). A soluble fragment region was determined by amino acid sequence homology analysis between human Bst2 and mouse Damp1. As a result, a vector expressing the soluble Bst2 fragment of SEQ ID No. 5 and another vector expressing the soluble Damp1 fragment of SEQ ID No. 6 were obtained.

### EXAMPLE 3: Real-time quantitative RT-PCR

Intracellular expression levels of specific genes were analyzed by real-time quantitative RT-PCR using ABI Prism 7900HT (Applied Biosystems, Foster City, CA) and a SYBR-Green assay kit. Primers and probes used were designed using Primer Express software (Applied Biosystems).

10 ng of single-stranded cDNA was placed in a reaction tube and subjected to multiplex TaqMan PCR (50 µl) using the TaqMan Universal PCR Master Mix. The relative amount of target cDNA was calculated using the comparative cycle threshold (CT) method. PCR products were analyzed by agarose gel electrophoresis.

The relative levels of a specific gene A were expressed as a change compared to a control sample (untransfected cells). All values were obtained using a 2-CT (Cₜ₁-Cₜ₀, Cₜ₁=C_{t1A}-C_{t1B}, Cₜ₀=C_{t0A}-Ct0B) calculation method relative to a normalization gene B (human GAPDH gene) in transfected cells. Each value was obtained from each sample in triplicate. The above experiments were carried out to quantify the expression of the Bst2 gene and interleukin-2.

### EXAMPLE 4: Expression and purification of soluble Bst2 protein fragment or Damp1 protein fragment

In order to express the above-prepared soluble Bst2 protein fragment or Damp1 protein fragment, a vector DNA was transiently or permanently introduced into specific animal cells. Transient transfection was performed by calcium phosphate (CaPO₄) precipitation, as follows. 24 hrs before transfection, 7x10⁶ 293T cells (ATCC) were seeded onto a 150-mm cell culture plate and cultured. One hour before transfection, the culture medium was exchanged with IMDM medium (Cambrex) supplemented with 2% fetal bovine serum (FBS; GIBCO-BRL). 1.5 ml of TE buffer (1 mM Tris, 0.1 mM EDTA, pH 8.0) containing 75 µg of DNA and 250 mM calcium was mixed with 1.5 ml of HEPES buffer (50 mM HEPES, 140 mM NaCl, 1.4 mM Na₂HPO₄ pH 7.05), was incubated for about 1 min at room temperature, and was applied to the pre-cultured cells. The cells were incubated in a CO₂ incubator at 37°C for 6 hrs. After the DNA/calcium solution was removed, the cells were refed with serum-free medium and further cultured for 72 hrs or longer, and the culture medium was then recovered. Separately, a permanent cell line was established using lipofectamine and dihydrofolate reductase as a selectable marker, as follows. 48 hrs before transfection, 1.35x10⁶ CHO-DUKX-B11 (dhfr⁻) cells (ATCC) were seeded onto a 100-mm cell culture plate and cultured in IMDM medium complemented with 10% FBS. 0.6 ml of serum-free IMDM medium containing 18 µg of DNA was mixed with 0.6 ml of serum-free IMDM medium containing 54 µl of Lipofectamine 2000 (Invitrogen), and was incubated at room temperature for 45 min. The DNA/lipofectamine mixture was supplemented with 8.8 ml of serum-free IMDM medium and applied to the pre-cultured cells. The cells were incubated in a CO₂ incubator at 37°C for 6 hrs. The medium was exchanged with a selection medium, 10% dialyzed FBS-containing IMDM medium. To analyze the transiently expressed protein, the cells were further cultured for 72 hrs or longer. The medium was then recovered and passed through a 0.2-µm filter (Millipore). The produced Bst2 soluble fragment protein was analyzed by immunoblotting using anti-Bst2 polyclonal antibody (Roche) or anti-histidine antibody (Roche).

For large-scale expression and purification of the soluble Bst2 protein fragment or Damp1 protein fragment, host cell lines into which a Bst2 or Damp1 expression vector was stably introduced were selected as production cell lines, as follows. CHO cells deleted in dihydrofolate reductase (DHFR) gene were transfected with an expression vector. Since the expression vector carried a dhfr gene, dihydrofolate reductase was used as a selectable marker. After 48 hrs, the transfected CHO cells were seeded onto a 96-well cell culture plate in a density of 1×10³ cells/well and cultured in a medium containing 20 nM methotrexate (MTX) to amplify the DHFR gene. After two weeks, the medium was recovered and subjected to ELISA using anti-Bst2 antibody to compare clones for the expression levels of Bst2 soluble fragment protein. Clones exhibiting high expression levels were selected and exposed to gradually increased concentrations of MTX up to 300 nM to complete gene amplification. Thereafter, the medium was collected from each clone and subjected to ELISA and immunoblotting in order to finally select a production cell line exhibiting the highest protein expression levels. Since the Bst2 soluble fragment protein was produced in the culture medium under serum-free conditions, the expressed protein was purified from the collected medium using the six-histidine tag added to the C-terminus. Protein purification was performed by NTA chelating chromatography using a column, NTA chelating agarose CL-6B (Peptron Inc.). The purity of the purified protein was analyzed by electrophoresis and ELISA, and the amount of the purified protein was determined by a BCA method (Biorad, USA) and UV spectrophotometry.

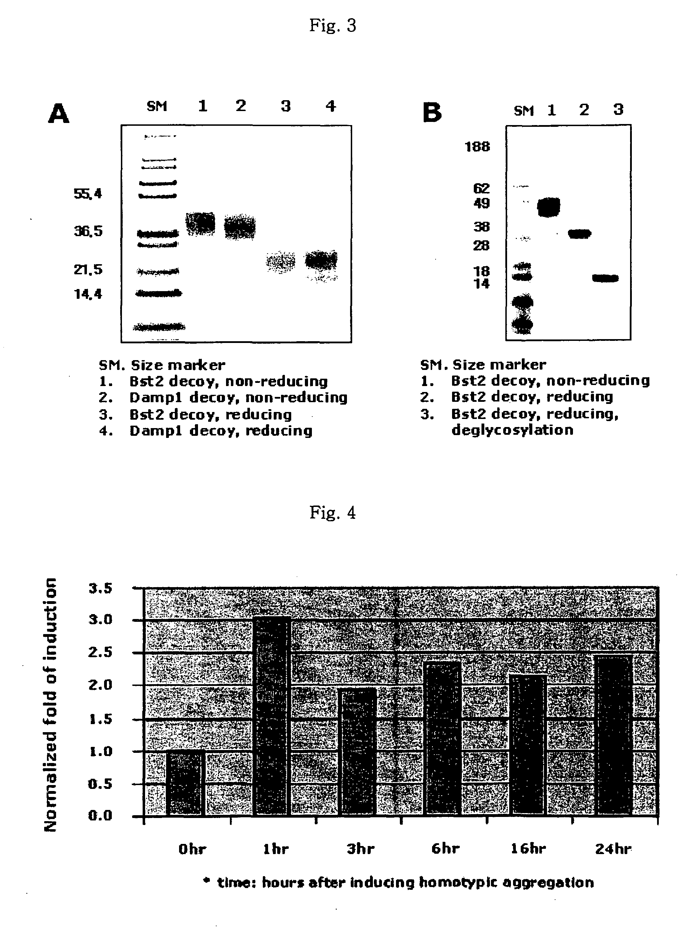

The human Bst2 soluble fragment and the mouse Damp1 soluble fragment, purified as described above, were analyzed by 4-20% SDS-PAGE (Fig. 3, panel A). The treatment of 1% dithiothreitol (DTT) and N-glycosidase F (Sigma) resulted in the Bst2 soluble fragment being a dimeric glycoprotein (Fig. 3, panel B). The results of the following examples were obtained using, among the prepared soluble fragments, a soluble Bst2 protein fragment having the amino acid sequence of SEQ ID No. 5 and a soluble Damp1 protein fragment having the amino acid sequence of SEQ ID No. 6.

### EXAMPLE 5: Evaluation of the effect of Bst2 protein on homotypic aggregation of U937 cells

### 5-1: Change in expression levels of Bst2 during aggregation of U937 cells

Expression levels of Bst2 protein were examined during aggregation of human U937 monocytic cells. 1x10⁶ U937 cells were treated with PMA (2 ng/ml) and LPS (10 µg/ml) for 24 hrs to induce homotypic cell aggregation of U937 cells, and were observed for the degree of homotypic cell aggregation under a phase-contrast inverted microscope (Olympus 1X71, state, USA). To determine the degree of cell aggregation, the size of formed cell aggregates was measured as pixel intensity, using Adobe's Photoshop software, version 7.0. The standard deviation values shown in drawings were calculated from mean values of six randomly selected aggregates. Thereafter, all used cells were recovered, and total RNA was isolated and subjected to RT-PCR using a set of primers of SEQ ID Nos. 16 and 17 to assess Bst2 expression levels.
Sense oligomer: 5'-TTTTCTCTTCTCAGTCTC-3' (SEQ ID No. 16)
Antisense oligomer: 5'-GCATCTACTTCGTATGAC-3' (SEQ ID No. 17)

One hour after U937 cells were treated with PMA and LPS to induce homotypic aggregation, intracellular Bst2 expression increased by about three times. This increased level was maintained for 24 hrs. These results indicate that Bst2 gene expression increases during homotypic aggregation of U937 cells (Fig. 4).

### 5-2: The effect of Bst2 protein on homotypic aggregation of U937 cells

In order to determine whether the increased expression of Bst2 gene is essential for the homotypic aggregation of U937 cells, cell aggregation was assessed when Bst2 protein was overexpressed.

1x10⁶ U937 cells, which had been cultured under the aforementioned conditions, were seeded onto a 96-well cell culture plate (NUNC) and treated with PMA (2 ng/ml, Calbiochem) and LPS (10 µg/ml, Calbiochem) for 24 hrs. The cells were then observed for the degree of homotypic cell aggregation under a phase-contrast inverted microscope (Olympus 1X71, state, USA).

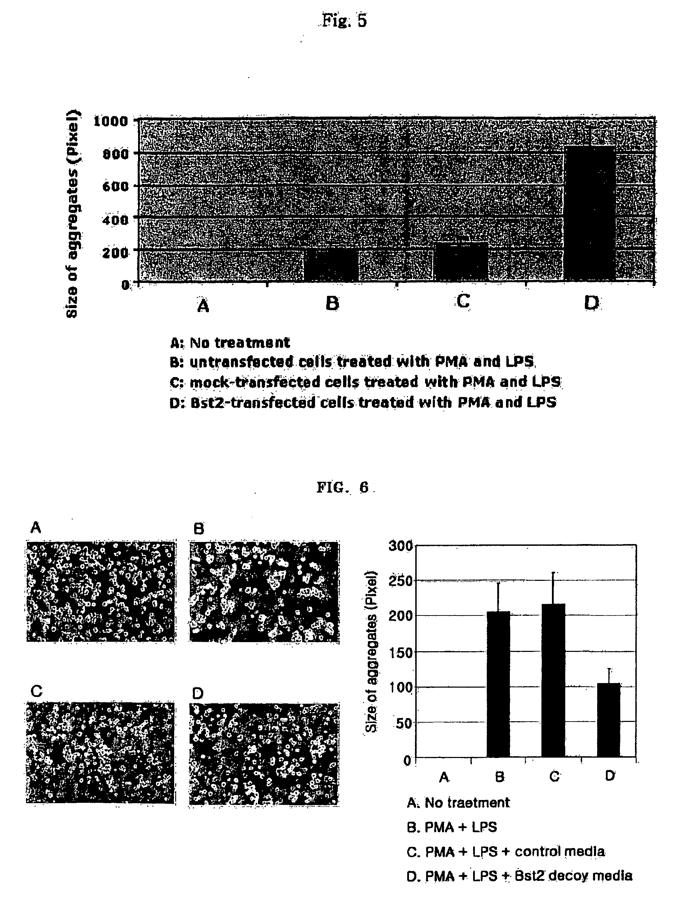

Bst2 protein itself did not induce aggregation of U937 cells, whereas the PMA/LPS treatment stimulated homotypic aggregation of U937 cells. Also, the transient overexpression of Bst2 increased homotypic aggregation of U937 cells by about four times (Fig. 5). These results indicate that Bst2 expression is a requisite condition for intercellular adhesion.

### 5-3: Inhibition of homotypic aggregation of U937 cells using Bst2 soluble fragment

In order to confirm whether the increased expression of Bst2 gene is essential for homotypic aggregation of U937 cells, cell aggregation was assessed when the action of Bst2 protein was suppressed.

U937 cells were pretreated with PMA and LPS to induce cell aggregation, and were treated with serial dilutions of medium (decoy medium) containing a Bst2 soluble fragment transiently expressed in CHO-S cells. The Bst2 soluble fragment was found to decrease U937 cell aggregation induced by PMA and LPS by 50% in comparison with the culture (control medium) of CHO-S cells not expressing the Bst2 soluble fragment (Fig. 6). These results indicate that the Bst2 soluble fragment inhibits homotypic aggregation of U937 cells.

### EXAMPLE 6: Evaluation of the effect of Bst2 protein on heterotypic aggregation between two different cell types

### 6-1: Inhibition of aggregation between U937 and HUVEC cells using Bst2 soluble fragment

HUVEC cells (1-5x10⁴ cells/ml) were seeded onto a 12-well cell culture plate. After one day, the medium was exchanged with a low-serum medium containing 0.5% FBS, and the cells were pretreated with interferon-gamma (IFN-γ; Calbiochem) in a final concentration 10 ng/ml for 24 hrs. Then, the pretreated HUVEC cells were co-cultured with U937 cells (2x10⁶ cells/ml, 500µl) at 37°C for 4 hrs. The co-culture was washed with phosphate buffer three or four times, and the remaining cells were fixed with 4% paraformaldehyde and microscopically observed.

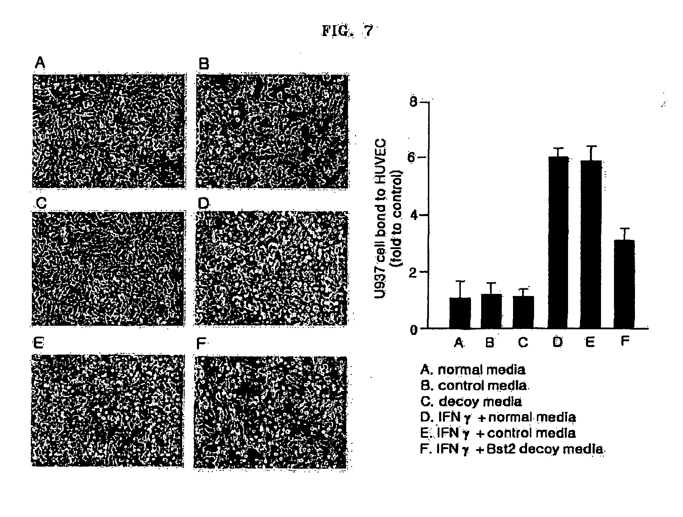

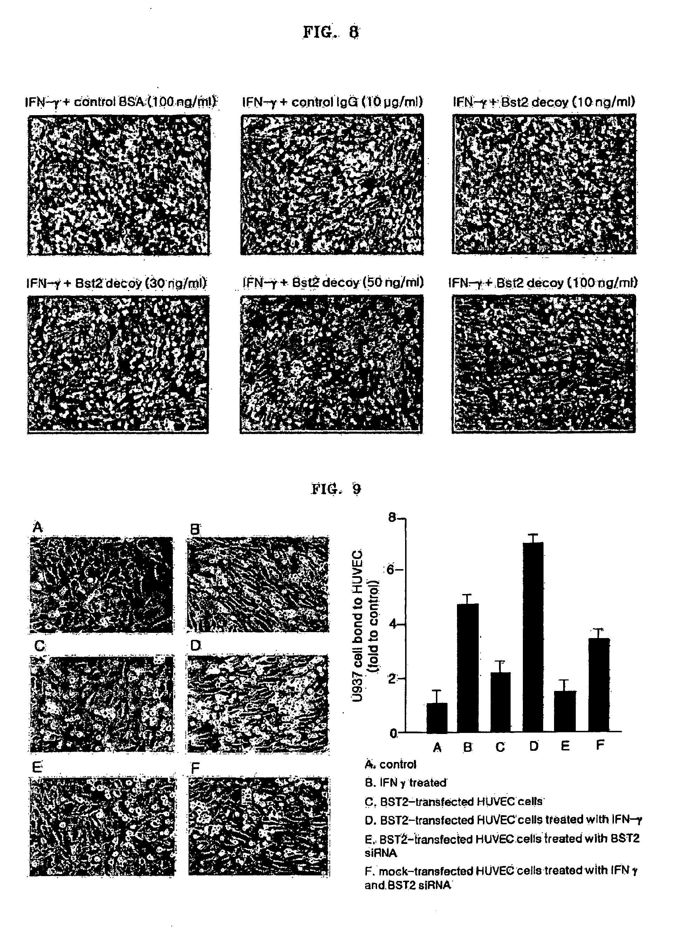

HUVEC cells not pretreated with IFN-γ did not bind to U937 cells. In contrast, IFN-γ-treated HUVEC cells bound to U937 cells and formed heterotypic cell aggregation. HUVEC cells which were pretreated with IFN-γ and were treated with a Bst2 soluble fragment protein-containing medium exhibited decreased aggregation with U937 cells. The treatment of a basic medium or albumin did not affect cell aggregation (Fig. 7). In Fig. 7, a "normal medium" indicates a FBS-containing general medium, and a "control medium" indicates a culture fluid of cells not expressing a Bst2 soluble fragment protein. In addition, the heterotypic cell aggregation was inhibited in such a manner of being dependent on concentrations of the Bst2 soluble fragment (Fig. 8).

### 6-2: Inhibition of aggregation between U937 and HUVEC cells using Bst2 siRNA

Various siRNA molecules acting in a Bst2-specific manner were constructed (QIAGEN). A total of 23 siRNA molecules specific to Bst2 were constructed. Each siRNA molecule consisted of an antisense RNA strand, complementary to Bst2 mRNA encoded by any one of the sequences of SEQ ID Nos. 18 to 40, and a sense RNA strand complementary to the antisense RNA strand.

The test results below were obtained using siRNA consisting of an antisense RNA strand, complementary to Bst2 mRNA encoded by the sequence of SEQ ID No. 38, and a sense RNA strand complementary to the antisense RNA strand.

HUVEC cells were transfected with a siRNA molecule (HPP grade, QIAGEN) consisting of a sense strand and an antisense strand under the same conditions as described above, were treated with IFN-γ, and were assessed for aggregation with U937 cells.
Target sequence: 5'-AAGCGTGAGAATCGCGGACAA-3' (SEQ ID No. 38)
Sense oligomer: 5'-r(UUGUCCGCGAUUCUCACGC)d(TT)-3'
Antisense oligomer: 5'-r(GCGTGAGAATCGCGGACAA)d(TT)-3'

Non-specific siRNA did not affect the heterotypic cell aggregation. In contrast, Bst2 gene-specific siRNA, unlike a control, completely inhibited aggregation between U937 cells and HUVEC cells (Fig. 9).

In order to determine whether Bst2 protein affects the adhesion of HUVEC cells to U937 cells, Bst2 protein was transiently overexpressed in HUVEC cells by transfection.

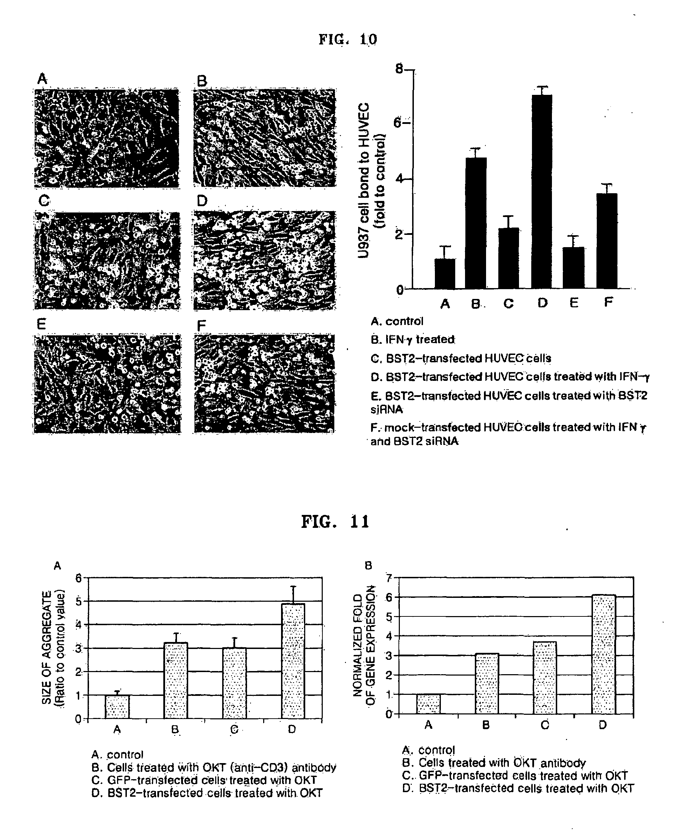

Quantitative analysis of heterotypic cell aggregation resulted in the finding that the increased expression of Bst2 protein increased aggregation by 50% or higher compared to a single treatment of IFN-γ. When Bst2 protein-overexpressed HUVEC cells were treated with siRNA of the Bst2 gene, heterotypic cell aggregation increased by Bst2 overexpression was inhibited again. These results indicate that Bst2 protein expression is important for heterotypic cell aggregation (Fig. 10).

### EXAMPLE 7: Evaluation of the effect of Bst2 protein on homotypic aggregation of T lymphocytes and activity of the aggregation

### 7-1: The effect of Bst2 overexpression on homotypic aggregation of T lymphocytes and IL-2 production

Human Jurkat T cells were induced to form homotypic cell aggregation and activated, as follows.

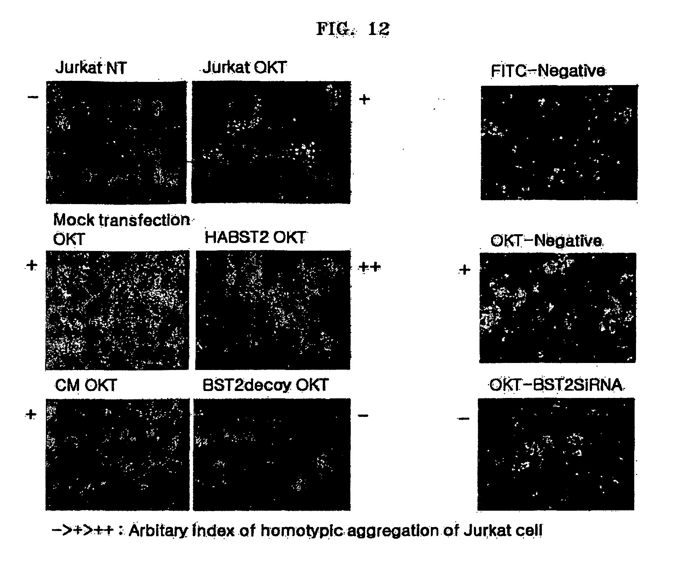

When Jurkat cells (5x10⁵ cells/ml) were incubated with anti-CD3 monoclonal antibody (OKT3: 10 µg/ml, BD Pharmingen) at 4°C for 20 min and then with anti-mouse immunoglobulin polyclonal antibody (25 µg/ml, Zymed) 37°C for 1 hr, cell aggregation occurred, and the cells were activated and induced to produce interleukin-2 (IL-2) (Figs. 11 and 12). According to the same method, when green fluorescent protein (GFP) overexpression was induced, there was no effect. In contrast, when Jurkat cells were transfected with a Bst2-overexpressing vector and were induced to activate, homotypic cell aggregation increased by 5% or higher (Fig. 11, panel A). IL-2 mRNA levels upon T cell activation were measured by real-time RT-PCR (Example 3). IL-2 mRNA expression was elevated by about two times under Bst2 overexpression in comparison with GFP overexpression (Fig. 11, panel B).

### 7-2: The effect of Bst2 soluble fragment and Bst2 siRNA on homotypic aggregation of T lymphocytes and IL-2 production

Jurkat cells were pretreated with a Bst2 soluble fragment 30 min before activation, were activated using anti-CD3 monoclonal antibody, and were evaluated for inhibition of cell aggregation. The cells were treated with a relative amount of serial dilutions of an animal cell culture fluid containing a Bst2 soluble fragment. The size aggregates was represented as a ratio to the size of aggregates of a non-treatment group.

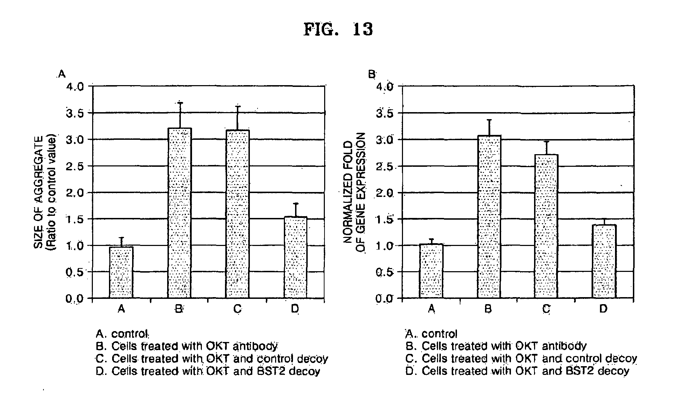

The Bst2 soluble fragment pretreatment under the activation condition resulted in a 50% decrease in aggregation of Jurkat cells. In addition, the 3-fold increased expression of IL-2 by Jurkat cell activation was decreased again to the basal level by the Bst2 soluble fragment treatment (Figs. 12 and 13).

### EXAMPLE 8: Evaluation of the action of Bst2 soluble fragment in a mouse model of asthma

### 8-1: Asthma induction in mice

A mouse model of asthma was prepared by sensitizing mice (BALB/c, 8 weeks) with ovalbumin. In detail, mice were initially sensitized for five continuous days by intranasal injection of ovalbumin. After three weeks, mice were intranasally sensitized again with ovalbumin for five continuous days. One week after the secondary sensitization, mice were challenged intranasally with ovalbumin three times every 24 hrs to induce asthma. Herein, a Bst2 soluble fragment was intravenously injected into mice 30 min before the last sensitization with ovalbumin, and was injected to mice 30 min before the first and the third injection of ovalbumin. Three days after the last injection, serum samples, lung tissues, and the like were collected from mice.

### 8-2: Bst2 soluble fragment-induced changes in the number of sedimented immune cells

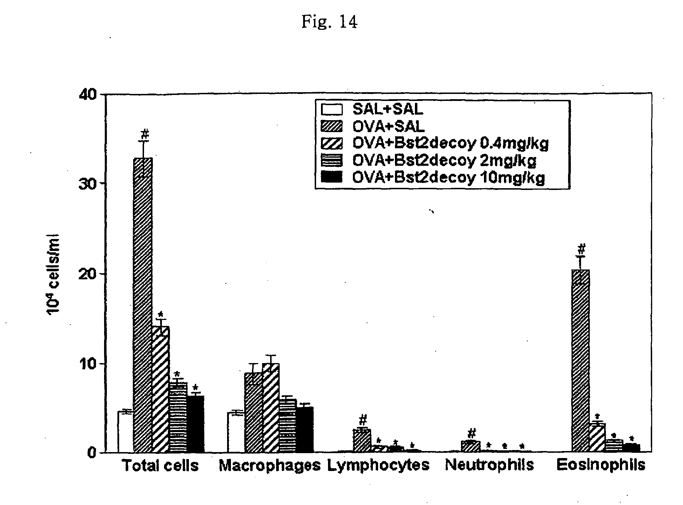

When a Bst2 or Damp1 soluble fragment was injected in a dose of 10 mg/kg into a mouse model of asthma which was induced by sensitization and challenge with ovalbumin, changes in the number of neutrophils, eosinophils, marcrophages, lymphocytes and other cell types were assessed. Three days after the last injection of ovalbumin, mice were sacrificed, and the chest was incised to expose the lung and other organs. After the trachea was dissected at its upper part, a cannula was carefully inserted into the trachea, and bronchoalvelar lavage was performed with physiological saline prewarmed to 37°C. The lavage fluids were collected, pooled, and centrifuged at 4°C. The sedimented cells were used for total cell counting or differential cell counting after being stained. The cell counting was performed with a hemocytometer under a microscope. In bronchoalvelar lavage fluid collected 72 hrs after sensitization with ovalbumin, the total number of cells, including neutrophils, eosinophils, marcrophages and lymphocytes, increased in comparison with a control pretreated with physiological saline. When ovalbumin-sensitized mice were treated with a Bst2 soluble fragment, the total cell number and the number of each cell type (neutrophils, eosinophils and lymphocytes) remarkably decreased in bronchoalvelar lavage fluid (Fig. 14).

### 8-3: The effect of Bst2 soluble fragment on cytokine production

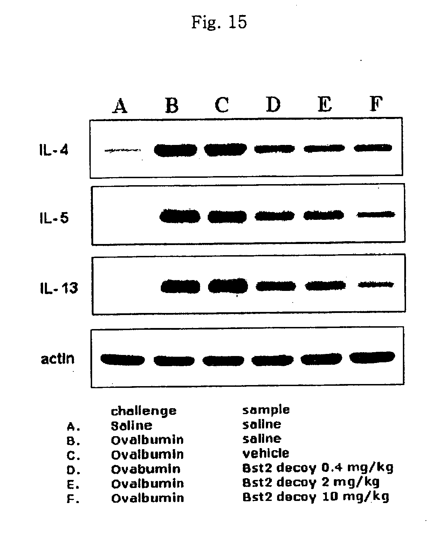

When a Bst2 or Damp1 soluble fragment was injected into a mouse model of asthma which was induced by sensitization and challenge with ovalbumin, expression levels of cytokines (interleukin-4 (IL-4), interleukin-5 (IL-5) and interleukin-13 (IL-13)) were measured, as follows. After bronchoalvelar lavage, lung tissues were excised from mice, and proteins were isolated from the lung tissues. Cytosolic proteins were isolated using lysis buffer containing NP-40. The isolated proteins were separated on a SDS-PAGE gel, and were transferred onto a PVDF membrane by a wet transfer method. The blot was incubated in a 1:1000 dilution of each several primary antibodies (anti-IL-4 antibody (Setotec Inc.), anti-IL-5 antibody (Santa Cruz Inc.), anti-IL-13 antibody (R&D Inc.), and anti-actin antibody (Sigma Inc.)). The bound primary antibodies were detected with a HRP-conjugated secondary antibody (anti-rabbit HRP-conjugated IgG) using ECL reagent. The levels of cytokines, such as IL-4, IL-5 and IL-13, were found to increase in the lung tissue of mice with asthma induced by sensitization and challenge with ovalbumin. Also, when ovalbumin-sensitized asthmatic mice were injected with a Bst2 decoy protein, cytokine levels decreased with increasing doses of the decoy protein. These results indicate that the Bst2 decoy protein has a therapeutic effect on asthma (Fig. 15).

### EXAMPLE 9: Evaluation of functional similarity between human Bst2 protein and mouse Damp1 protein

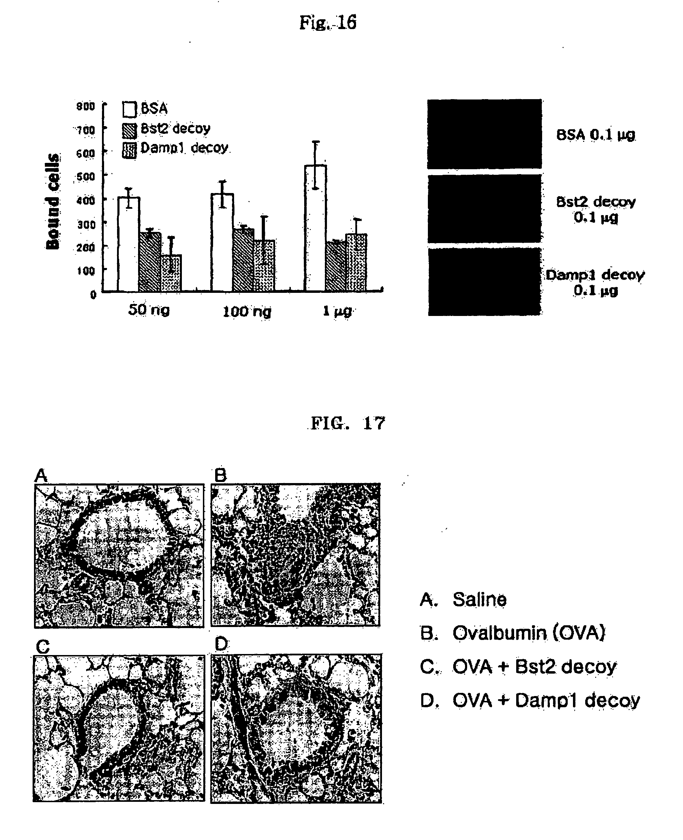

There is an about 35% amino acid sequence similarity between human Bst2 protein and mouse Damp1 protein. In this regard, it was examined that the two proteins interact with each other *in vivo.* Human Bst2 and mouse Damp1 proteins were examined for an inhibitory effect on adhesion between IFN-γ-treated HUVEC cells and U937 cells according to the same method as in Example 5. The treatment of bovine serum albumin (BSA) as a control protein resulted in no change in the number of U937 cells bound to HUVEC cells in comparison with the case of being treated with only culture medium. When cells were treated with human Bst2 decoy protein and mouse Damp1 decoy protein, the intercellular adhesion between HUVEC cells and U937 cells were inhibited in a dose-dependent manner (Fig. 16). Separately, the lung tissue collected in Example 8 from asthmatic mice three days after asthma induction was fixed in 10% formaldehyde, embedded in paraffin and sectioned. The paraffin sections were stained with hematoxylin and eosin. Neutrophils, eosinophils, marcrophages, lymphocytes and other cell types were recruited to and filled the alveolar tissue of ovalbumin-sensitized asthmatic mice, and the airway epithelial tissue was thickened and covered with mucous secretions and cellular debris (Fig. 17). When asthmatic mice were treated with a mouse Damp1 soluble fragment or human Bst2 soluble fragment, the number of Neutrophils, eosinophils, marcrophages, lymphocytes and other cell types, recruited into the alveolar tissue, was greatly reduced, and no histopathological abnormality was observed in the alveolar tissue like that of non-asthmatic mice (treated with physiological saline). These results indicate that a mouse Damp1 soluble fragment has an asthma-inhibiting effect comparable to that of a human Bst2 soluble fragment protein.

### EXAMPLE 10: Preparation of anti-Bst2 polyclonal antibody

The purified Bst2 and Damp1 decoy proteins expressed in CHO-S cells were mixed with a Ribi adjuvant at a ratio of 1:1, and were injected into rabbits with time intervals of two weeks. During immunization, blood samples were collected and examined for antibody production. After three immunizations, serum samples were obtained from rabbits. Anti-Bst2 polyclonal antibody was purified by affinity chromatography using a column in which Bst2 protein was bound to an immobilized support.

### EXAMPLE 11: Preparation of PEG-conjugated forms for improvement of metabolism of Bst2 soluble fragment

### 11-1. Preparation of PEG-conjugated forms

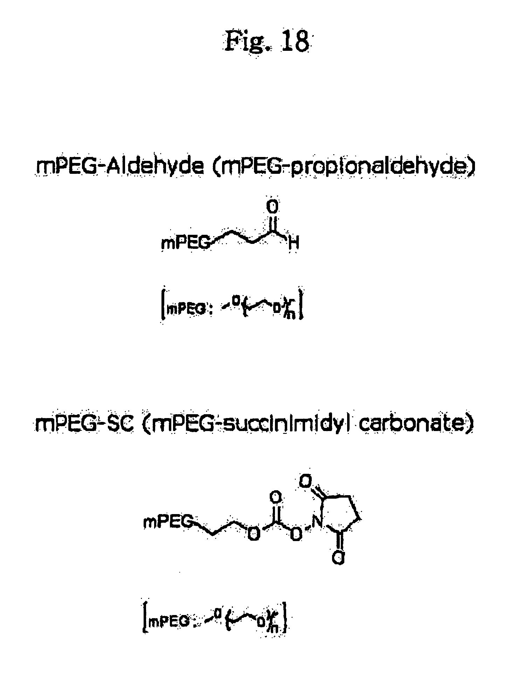

PEG conjugation was carried out by two types of PEG: (1) aldehyde PEG and (2) succinimidyl carbonate PEG (Fig. 18). First, aldehyde PEG conjugation was carried out as follows. 1 mg of Bst2 soluble fragment protein was dialyzed in 0.1 M phosphate buffer (pH 7.5), and was mixed with a 30-fold molar ratio of (mPEG12000-OCH₂COGly-Gly)₂(2,4-diamino butylic acid)-PEG'-NHS, followed by incubation at room temperature of 2 hrs with agitation. Separately, for carbonate PEG conjugation, 1 mg of Bst2 soluble fragment protein was dialyzed in 0.1 M phosphate buffer (pH 5.0), and was mixed with a 20-fold molar ratio of succinimidyl carbonate PEG, followed by incubation at room temperature of 2 hrs with agitation. After the reaction was completed, PEG-conjugated Bst2 soluble fragments were isolated and purified using a size exclusion column (Superdex-200, Pharmacia), and were dialyzed in 50 mM phosphate buffer (pH 7.4).

### 11-2. The enhancing effect of PEG-conjugated forms on in vivo stability of Bst2 soluble fragment

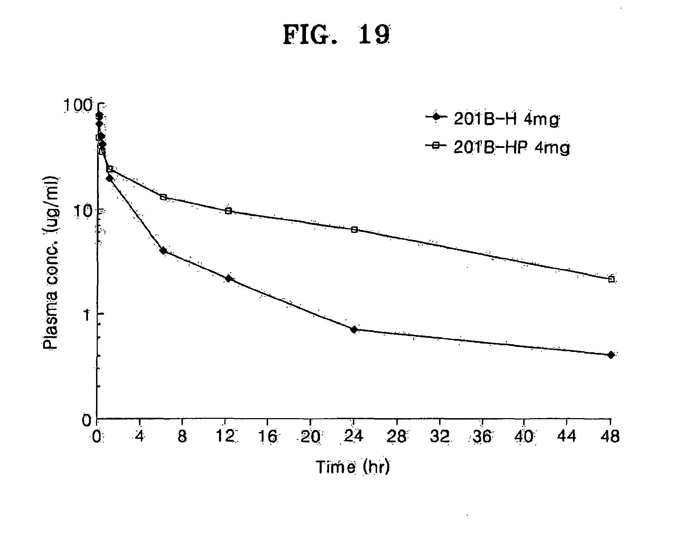

The PEG-conjugated forms of Bst2 soluble fragment, prepared in Example 11-1, were injected into the tail vein of 7 week-old male Sprague-Dawley rats in a dose of 0.4 to 2 mg/kg. A negative control group was injected with an equal dose of physiological saline. Also, an equal dose of Bst2 soluble fragment protein was used as a positive control. Blood samples were collected before drug administration, and 2 min, 5 min, 10 min, 30 min, 1 hr, 2 hrs, 6 hrs, 12 hrs and 24 hrs after drug administration from the jugular vein using a cannula. The collected blood samples were analyzed by ELISA. A 96-well plate was coated with an anti-Bst2 soluble fragment antibody (100 ng/ml in PBS) at 4°C for 8 hrs or longer, and was blocked with albumin in PBS at 37°C for 2 hrs. The plate was reacted with a proper dilution of rat serum or Bst2 soluble fragment (standard sample) at 37°C for 2 hrs. The plate was then reacted with a monoclonal antibody (mAb conjugated with horseradish peroxidase, Roche Inc.) recognizing the histidine tag added to the C-terminus of Bst2 soluble fragment at 37°C for 2 hrs. After being well washed, the plate was treated with a substance of peroxidase, and absorbance was measured at 450 nm. Quantization of the PEG-conjugated Bst2 soluble fragments present in blood was performed using the standard samples (Fig. 19). In Fig. 19, "201B-H" indicates a human Bst2 soluble fragment sample, and "201B-HP" indicates an aldehyde PEG-conjugated human Bst2 soluble fragment sample.

### EXAMPLE 12: Expression and distribution of Bst2 in inflammation-associated diseases

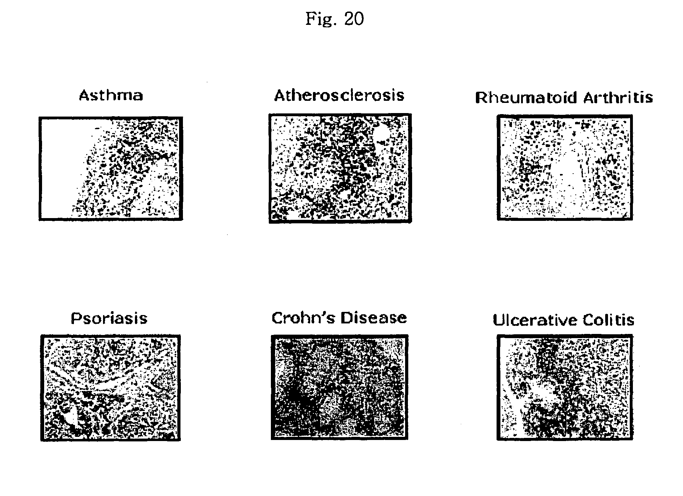

Expression levels of Bst2 protein were examined in inflammation-associated diseases including asthma, atherosclerosis, rheumatoid arthritis, psoriasis, Crohn's disease and ulcerative colitis. A paraffin block of the lung tissue, prepared by fixing the lung tissue in 10% formaldehyde and embedding the tissue in paraffin, was sectioned into a thickness of 1.5 µm, and was mounted onto glass slides. The slides were stained with hematoxylin and eosin to investigate the changes in the lung tissue according to allergen and drug administration. Histostaining was performed with the polyclonal antibody prepared in Example 10. As a result, compared to the normal tissue, Bst2 protein was overexpressed in inflammation-associated diseases, and was expressed in immune cells, vascular endothelial cells and other cell types (Fig. 20).

### Industrial Applicability

As described hereinbefore, the present invention provides antagonists of Bst2 for used in the treatment of an inflammation-associated disease mediated by Bst2, namely asthma.

## Claims

1. A pharmaceutical composition for use in preventing or treating an inflammatory disease, comprising as an active ingredient:
(i) a fragment of the Bst2 protein comprising the amino acid sequence of SEQ ID NO: 2 having an inflammation-suppressing effect, wherein the fragment of the Bst2 protein has a deletion of the whole or a portion of an intracellular domain thereof and comprises the amino acid sequence of SEQ ID NO: 5; or
(ii) a variant of the protein of (i) **characterized by** having an amino acid sequence different from the sequence of the protein of (i) in one or more amino acid residues by a deletion, an insertion, a non-conservative or conservative substitution or a combination thereof, wherein the variant has an inflammation-suppressing effect,
wherein the inflammatory disease is asthma.

2. The pharmaceutical composition for use in preventing or treating an inflammatory disease according to claim 1, wherein the non-conservative or conservative substitution is a substitution from Ala to Ser, from Ser to Ala, from Val to Ile, from Ile to Val, from Asp to Glu, from Glu to Asp, from Thr to Ser, from Ser to Thr, from Ala to Gly, from Gly to Ala, from Ala to Thr, from Thr to Ala, from Ser to Asn, from Asn to Ser, from Ala to Val, from Val to Ala, from Ser to Gly, from Gly to Ser, from Thy to Phe, or from Phe to Thy.

3. The pharmaceutical composition for use in preventing or treating an inflammatory disease according to claim 1 or 2, wherein the Bst2 protein or the fragment thereof is modified by a non-peptide polymer.

4. The pharmaceutical composition for use in preventing or treating an inflammatory disease according to claim 3, wherein the non-peptide polymer is selected from the group consisting of polyethylene glycol, polypropylene glycol,co-poly(ethylene/propylene) glycol, polyoxyethylene, polyurethane, polyphosphazene, polysaccharide, dextran, polyvinyl alcohol, polyvinyl pyrrolidones, polyvinyl ethyl ether, polyacryl amide, polyacrylate, polycyanoacylate, lipid polymer, chitins, hyaluronic acid, heparin and combinations thereof.

## Patentansprüche

1. Pharmazeutische Zusammensetzung für die Verwendung bei der Vorbeugung oder Behandlung einer entzündlichen Erkrankung, als Wirkstoff umfassend:
(i) ein Fragment des Bst2-Proteins umfassend die Aminosäuresequenz von SEQ ID NO: 2, welches einen die Entzündung unterdrückenden Effekt aufweist, wobei das Fragment des Bst2-Proteins eine Deletion der ganzen oder eines Teils der intrazellulären Domäne davon aufweist und die Aminosäuresequenz von SEQ ID NO: 5 umfasst; oder
(ii) eine Variante des Proteins nach (i), welche dadurch charakterisiert ist, dass sie eine Aminosäuresequenz aufweist, welche sich von der Sequenz nach (i) in einem oder mehreren Aminosäureresten durch eine Deletion, eine Insertion, eine nicht-konservative oder konservative Substitution, oder einer Kombination davon unterscheidet, wobei die Variante einen die Entzündung unterdrückenden Effekt aufweist,
wobei die entzündliche Erkrankung Asthma ist.

2. Die pharmazeutische Zusammensetzung für die Verwendung bei der Vorbeugung oder Behandlung einer entzündlichen Erkrankung nach Anspruch 1, wobei die nicht-konservative oder konservative Substitution eine Substitution ist von Ala nach Ser, von Ser nach Ala, von Val nach Ile, von Ile nach Val, von Asp nach Glu, von Glu nach Asp, von Thr nach Ser, von Ser nach Thr, von Ala nach Gly, von Gly nach Ala, von Ala nach Thr, von Thr nach Ala, von Ser nach Asn, von Asn nach Ser, von Ala nach Val, von Val nach Ala, von Ser nach Gly, von Gly nach Ser, von Thy nach Phe, oder von Phe nach Thy.

3. Die pharmazeutische Zusammensetzung für die Verwendung bei der Vorbeugung oder Behandlung einer entzündlichen Erkrankung nach Anspruch 1 oder 2, wobei das Bst2-Protein oder das Fragment davon mittels eines nicht-peptidischen Polymers modifiziert ist.

4. Die pharmazeutische Zusammensetzung für die Verwendung bei der Vorbeugung oder Behandlung einer entzündlichen Erkrankung nach Anspruch 3, wobei das nichtpeptidische Polymer ausgewählt ist aus der Gruppe bestehend aus Polyethylenglykol, Polypropylenglykol, Co-poly(ethylen/propylen)-glykol, Polyoxyethylen, Polyurethan, Polyphosphazen, Polysaccharid, Dextran, Polyvinylalkohol, Polyvinylpyrrolidonen, Polyvinylethylether, Polyacrylamid, Polyacrylat, Polycyanoacylat, Lipidpolymer, Chitinen, Hyaluronsäure, Heparin und Kombinationen davon.

## Revendications

1. Composition pharmaceutique pour une utilisation dans le domaine de la prévention ou du traitement d'une maladie inflammatoire, comprenant, en tant qu'ingrédient actif :
(i) un fragment de la protéine Bst2 comprenant la séquence d'acide aminé de SEQ ID N° 2 ayant un effet anti-inflammatoire, où le fragment de la protéine Bst2 présente une délétion de l'intégralité ou d'une partie d'un domaine intracellulaire de celle-ci et comprend la séquence d'acide aminé de SEQ ID N° 5 ; ou
(ii) une variante de la protéine de (i) **caractérisée en ce qu'**elle présente une séquence d'acide aminé différente de la séquence de la protéine de (i) dans un ou plusieurs résidus d'acide aminé par le biais d'une délétion, d'une insertion, d'une substitution conservative ou non conservative ou d'une combinaison de celles-ci, le variant présentant une action anti-inflammatoire,
où la maladie inflammatoire est l'asthme.

2. Composition pharmaceutique pour une utilisation dans le domaine de la prévention ou du traitement d'une maladie inflammatoire selon la revendication 1, où la substitution non conservative ou conservative est une substitution de Ala en Ser, de Ser en Ala, de Val en Ile, de Ile en Val, de Asp en Glu, de Glu en Asp, de Thr en Ser, de Ser en Thr, de Ala en Gly, de Gly en Ala, de Ala en Thr, de Thr en Ala, de Ser en Asn, de Asn en Ser, de Ala en Val, de Val en Ala, de Ser en Gly, de Gly en Ser, de Thy en Phe, ou de Phe en Thy.

3. Composition pharmaceutique pour une utilisation dans le domaine de la prévention ou du traitement d'une maladie inflammatoire selon la revendication 1 ou 2, où la protéine Bst2 ou le fragment de celle-ci est modifié par un polymère non peptidique.

4. Composition pharmaceutique pour une utilisation dans le domaine de la prévention ou du traitement d'une maladie inflammatoire selon la revendication 3, où le polymère non peptidique est sélectionné parmi le groupe constitué de polyéthylène glycol, polypropylène glycol, co-poly(éthylène/propylène)glycol, polyoxyéthylène, polyuréthane, polyphosphazène, polysaccharide, dextran, alcool polyvinylique, polyvinylpyrrolidones, éther éthylique de polyvinyle, polyacrylamide, polyacrylate, polycyanoacylate, polymère lipidique, chitines, acide hyaluronique, héparine et des combinaisons de ceux-ci.


### Sequence Listing

<110> ISU Abxis Co.
<120> Molecules inhibiting intercellular adhesion
<150> KR10-2004-0108909
   <151> 2004-12-20
<160> 40
<170> KopatentIn 1.71
<210> 1
   <211> 543
   <212> DNA
   <213> Homo sapiens
<220>
   <221> CDS
   <222> (1)...(540)
   <223> full-length Bst2 cDNA sequence
<400> 1
   atg gca tct act tcg tat gac tat tgc aga gtg ccc atg gaa gac ggg      48
   Met Ala Ser Thr Ser Tyr Asp Tyr Cys Arg Val Pro Met Glu Asp Gly
   1               5                   10                  15
   gat aag cgc tgt aag ctt ctg ctg ggg ata gga att ctg gtg ctc ctg      96
   Asp Lys Arg Cys Lys Leu Leu Leu Gly Ile Gly Ile Leu Val Leu Leu
               20                  25                  30
   atc atc gtg att ctg ggg gtg ccc ttg att atc ttc acc atc aag gcc      144
   Ile Ile Val Ile Leu Gly Val Pro Leu Ile Ile Phe Thr Ile Lys Ala
           35                  40                  45
   aac agc gag gcc tgc cgg gac ggc ctt cgg gca gtg atg gag tgt cgc      192
   Asn Ser Glu Ala Cys Arg Asp Gly Leu Arg Ala Val Met Glu Cys Arg
       50                  55                  60
   aat gtc acc cat ctc ctg caa caa gag ctg acc gag gcc cag aag ggc      240
   Asn Val Thr His Leu Leu Gln Gln Glu Leu Thr Glu Ala Gln Lys Gly
   65                  70                  75                  80
   ttt cag gat gtg gag gcc cag gcc gcc acc tgc aac cac act gtg atg      288
   Phe Gln Asp Val Glu Ala Gln Ala Ala Thr Cys Asn His Thr Val Met
                   85                  90                  95
   gcc cta atg gct tcc ctg gat gca gag aag gcc caa gga caa aag aaa      336
   Ala Leu Met Ala Ser Leu Asp Ala Glu Lys Ala Gln Gly Gln Lys Lys
               100                 105                 110
   gtg gag gag ctt gag gga gag atc act aca tta aac cat aag ctt cag      384
   Val Glu Glu Leu Glu Gly Glu Ile Thr Thr Leu Asn His Lys Leu Gln
           115                 120                 125
   gac gcg tct gca gag gtg gag cga ctg aga aga gaa aac cag gtc tta      432
   Asp Ala Ser Ala Glu Val Glu Arg Leu Arg Arg Glu Asn Gln Val Leu
       130                 135                 140
   agc gtg aga atc gcg gac aag aag tac tac ccc agc tcc cag gac tcc      480
   Ser Val Arg Ile Ala Asp Lys Lys Tyr Tyr Pro Ser Ser Gln Asp Ser
   145                 150                 155                 160
   agc tcc gct gcg gcg ccc cag ctg ctg att gtg ctg ctg ggc ctc agc      528
   Ser Ser Ala Ala Ala Pro Gln Leu Leu Ile Val Leu Leu Gly Leu Ser
                   165                 170                 175
   gct ctg ctg cag         tga                                          543
   Ala Leu Leu Gln
               180
<210> 2
   <211> 180
   <212> PRT
   <213> Homo sapiens
<400> 2
   Met Ala Ser Thr Ser Tyr Asp Tyr Cys Arg Val Pro Met Glu Asp Gly
   1               5                   10                  15
   Asp Lys Arg Cys Lys Leu Leu Leu Gly Ile Gly Ile Leu Val Leu Leu
               20                  25                  30
   Ile Ile Val Ile Leu Gly Val Pro Leu Ile Ile Phe Thr Ile Lys Ala
           35                  40                  45
   Asn Ser Glu Ala Cys Arg Asp Gly Leu Arg Ala Val Met Glu Cys Arg
       50                  55                  60
   Asn Val Thr His Leu Leu Gln Gln Glu Leu Thr Glu Ala Gln Lys Gly
   65                  70                  75                  80
   Phe Gln Asp Val Glu Ala Gln Ala Ala Thr Cys Asn His Thr Val Met
                   85                  90                  95
   Ala Leu Met Ala Ser Leu Asp Ala Glu Lys Ala Gln Gly Gln Lys Lys
               100                 105                 110
   Val Glu Glu Leu Glu Gly Glu Ile Thr Thr Leu Asn His Lys Leu Gln
           115                 120                 125
   Asp Ala Ser Ala Glu Val Glu Arg Leu Arg Arg Glu Asn Gln Val Leu
       130                 135                 140
   Ser Val Arg Ile Ala Asp Lys Lys Tyr Tyr Pro Ser Ser Gln Asp Ser
   145                 150                 155                 160
   Ser Ser Ala Ala Ala Pro Gln Leu Leu Ile Val Leu Leu Gly Leu Ser
                   165                 170                 175
   Ala Leu Leu Gln
               180
<210> 3
   <211> 519
   <212> DNA
   <213> mouse
<220>
   <221> CDS
   <222> (1)..(516)
   <223> full-length Damp-1 cDNA sequence
<400> 3
   atg gcg ccc tct ttc tat cac tat ctg ccc gtg ccc atg gat gag atg 48
   Met Ala Pro Ser Phe Tyr His Tyr Leu Pro Val Pro Met Asp Glu Met
   1 5 10 15
   ggg ggg aag caa gga tgg ggc agc cac cgg cag tgg ctg ggg gcc gcg 96
   Gly Gly Lys Gln Gly Trp Gly Ser His Arg Gln Trp Leu Gly Ala Ala
   20 25 30
   atc ttg gtg gtc ctg ttc ggg gtt acc tta gtc atc ctg aca atc tac 144
   Ile Leu Val Val Leu Phe Gly Val Thr Leu Val Ile Leu Thr Ile Tyr
   35 40 45
   ttc gcc gtc aca gcg aac agc gtg gcc tgt aga gac ggg ttg cga gcg 192
   Phe Ala Val Thr Ala Asn Ser Val Ala Cys Arg Asp Gly Leu Arg Ala
   50 55 60
   cag gct gag tgc cgg aac acc acg cac ctg ttg cag cgc cag ctc acc 240
   Gln Ala Glu Cys Arg Asn Thr Thr His Leu Leu Gln Arg Gln Leu Thr
   65 70 75 80
   cgc acc cag gac agt ctg ctg cag gcc gag aca cag gca aac tcc tgc 288
   Arg Thr Gln Asp Ser Leu Leu Gln Ala Glu Thr Gln Ala Asn Ser Cys
   85 90 95
   aac ctg acc gtg gtg acc ctt cag gag tcc ctg gag aag aag gtg tct 336
   Asn Leu Thr Val Val Thr Leu Gln Glu Ser Leu Glu Lys Lys Val Ser
   100 105 110
   caa gcc ctg gag cag cag gcc cgc atc aag gag ctt gag aat gaa gtc 384
   Gln Ala Leu Glu Gln Gln Ala Arg Ile Lys Glu Leu Glu Asn Glu Val
   115 120 125
   acg aag ctg aac cag gag ctg gag aat ctg agg atc caa aag gag act 432
   Thr Lys Leu Asn Gln Glu Leu Glu Asn Leu Arg Ile Gln Lys Glu Thr
   130 135 140
   tct agc aca gtg cag gtg aac tct ggc agc tcc atg gtg gtc tcc agc 480
   Ser Ser Thr Val Gln Val Asn Ser Gly Ser Ser Met Val Val Ser Ser
   145 150 155 160
   cta ctg gtg ctc aaa gtg tca ctg ttc ctg ctc ttt tga 519
   Leu Leu Val Leu Lys Val Ser Leu Phe Leu Leu Phe
   165 170
<210> 4
   <211> 172
   <212> PRT
   <213> mouse
<400> 4
   Met Ala Pro Ser Phe Tyr His Tyr Leu Pro Val Pro Met Asp Glu Met
   1 5 10 15
   Gly Gly Lys Gln Gly Trp Gly Ser His Arg Gln Trp Leu Gly Ala Ala
   20 25 30
   Ile Leu Val Val Leu Phe Gly Val Thr Leu Val Ile Leu Thr Ile Tyr
   35 40 45
   Phe Ala Val Thr Ala Asn Ser Val Ala Cys Arg Asp Gly Leu Arg Ala
   50 55 60
   Gln Ala Glu Cys Arg Asn Thr Thr His Leu Leu Gln Arg Gln Leu Thr
   65 70 75 80
   Arg Thr Gln Asp Ser Leu Leu Gln Ala Glu Thr Gln Ala Asn Ser Cys
   85 90 95
   Asn Leu Thr Val Val Thr Leu Gln Glu Ser Leu Glu Lys Lys Val Ser
   100 105 110
   Gln Ala Leu Glu Gln Gln Ala Arg Ile Lys Glu Leu Glu Asn Glu Val
   115 120 125
   Thr Lys Leu Asn Gln Glu Leu Glu Asn Leu Arg Ile Gln Lys Glu Thr
   130 135 140
   Ser Ser Thr Val Gln Val Asn Ser Gly Ser Ser Met Val Val Ser Ser
   145 150 155 160
   Leu Leu Val Leu Lys Val Ser Leu Phe Leu Leu Phe
   165 170
<210> 5
   <211> 116
   <212> PRT
   <213> Homo sapiens
<220>
   <221> PEPTIDE
   <222> (1)..(116)
   <223> soluble Bst2 fragment
<400> 5
   Phe Thr Ile Lys Ala Asn Ser Glu Ala Cys Arg Asp Gly Leu Arg Ala
   1 5 10 15
   Val Met Glu Cys Arg Asn Val Thr His Leu Leu Gln Gln Glu Leu Thr
   20 25 30
   Glu Ala Gln Lys Gly Phe Gln Asp Val Glu Ala Gln Ala Ala Thr Cys
   35 40 45
   Asn His Thr Val Met Ala Leu Met Ala Ser Leu Asp Ala Glu Lys Ala
   50 55 60
   Gln Gly Gln Lys Lys Val Glu Glu Leu Glu Gly Glu Ile Thr Thr Leu
   65 70 75 80
   Asn His Lys Leu Gln Asp Ala Ser Ala Glu Val Glu Arg Leu Arg Arg
   85 90 95
   Glu Asn Gln Val Leu Ser Val Arg Ile Ala Asp Lys Lys Tyr Tyr Pro
   100 105 110
   Ser Ser Gln Asp
   115
<210> 6
   <211> 107
   <212> PRT
   <213> mouse
<220>
   <221> PEPTIDE
   <222> (1)..(107)
   <223> soluble Damp-1 fragment
<400> 6
   Thr Ile Tyr Phe Ala Val Thr Ala Asn Ser Val Ala Cys Arg Asp Gly
   1 5 10 15
   Leu Arg Ala Gln Ala Glu Cys Arg Asn Thr Thr His Leu Leu Gln Arg
   20 25 30
   Gln Leu Thr Arg Thr Gln Asp Ser Leu Leu Gln Ala Glu Thr Gln Ala
   35 40 45
   Asn Ser Cys Asn Leu Thr Val Val Thr Leu Gln Glu Ser Leu Glu Lys
   50 55 60
   Lys Val Ser Gln Ala Leu Glu Gln Gln Ala Arg Ile Lys Glu Leu Glu
   65 70 75 80
   Asn Glu Val Thr Lys Leu Asn Gln Glu Leu Glu Asn Leu Arg Ile Gln
   85 90 95
   Lys Glu Thr Ser Ser Thr Val Gln Val Asn Ser
   100 105
<210> 7
   <211> 24
   <212> DNA
   <213> Artificial Sequence
<220>
   <223> forward primer for the amplification of TPAsig_XhoI
<400> 7
   cgctcgagac agccatcatg gatg 24
<210> 8
   <211> 27
   <212> DNA
   <213> Artificial Sequence
<220>
   <223> reverse primer for the amplification of TPAsig+Bst2
<400> 8
   ggccttgatg gtgaagctgg gcgaaac 27
<210> 9
   <211> 23
   <212> DNA
   <213> Artificial Sequence
<220>
   <223> forward primer for the amplification of Bst2
<400> 9
   agcttcacca tcaaggccaa cag 23
<210> 10
   <211> 28
   <212> DNA
   <213> Artificial Sequence
<220>
   <223> reverse primer 1 for the amplification of Bst2
<400> 10
   gtgatgatgg tcctgggagc tggggtag 28
<210> 11
   <211> 32
   <212> DNA
   <213> Artificial Sequence
<220>
   <223> reverse primer 2 for the amplification of Bst2_XbaI
<400> 11
   gcagatcttc aatggtgatg gtgatgatgg tc 32
<210> 12
   <211> 27
   <212> DNA
   <213> Artificial Sequence
<220>
   <223> reverse primer for the amplification of TPAsig+Damp1
<400> 12
   cgctgtgacg gcgaagctgg gcgaaac 27
<210> 13
   <211> 24
   <212> DNA
   <213> Artificial Sequence
<220>
   <223> forward primer for the amplification of Damp1
<400> 13
   agcttcgccg tcacagcgaa cagc 24
<210> 14
   <211> 29
   <212> DNA
   <213> Artificial Sequence
<220>
   <223> reverse primer 1 for the amplification of Damp1
<400> 14
   gtgatgatga gagttcacct gcactgtgc 29
<210> 15
   <211> 31
   <212> DNA
   <213> Artificial Sequence
<220>
   <223> reverse primer 2 for the amplification of Damp1_XbaI
<400> 15
   gcagatcttc aatggtgatg gtgatgatga g 31
<210> 16
   <211> 18
   <212> DNA
   <213> Artificial Sequence
<220>
   <223> sense primer for quantification of Bst2 expression level
<400> 16
   ttttctcttc tcagtctc 18
<210> 17
   <211> 18
   <212> DNA
   <213> Artificial Sequence
<220>
   <223> antisense priemr for quantification of Bst2 expression level
<400> 17
   gcatctactt cgtatgac 18
<210> 18
   <211> 21
   <212> DNA
   <213> Homo sapiens
<220>
   <221> gene
   <222> (1)..(21)
   <223> sequence in Bst2 gene targeted by siRNA, starting from 41th base
<400> 18
   aagacgggga taagcgctat a 21
<210> 19
   <211> 21
   <212> DNA
   <213> Homo sapiens
<220>
   <221> gene
   <222> (1)..(21)
   <223> sequence in Bst2 gene targeted by siRNA, starting from 52th base
<400> 19
   aagcgctata agcttctgct g 21
<210> 20
   <211> 21
   <212> DNA
   <213> Homo sapiens
<220>
   <221> gene
   <222> (1)..(21)
   <223> sequence in Bst2 gene targeted by siRNA, starting from 61th base
<400> 20
   aagcttctgc tggggatagg a 21
<210> 21
   <211> 21
   <212> DNA
   <213> Homo sapiens
<220>
   <221> gene
   <222> (1)..(21)
   <223> sequence in Bst2 gene targeted by siRNA, starting from 81th base
<400> 21
   aattctggtg ctcctgatca t 21
<210> 22
   <211> 21
   <212> DNA
   <213> Homo sapiens
<220>
   <221> gene
   <222> (1)..(21)
   <223> sequence in Bst2 gene targeted by siRNA, starting from 139th base
<400> 22
   aaggccaaca gcgaggcctg c 21
<210> 23
   <211> 21
   <212> DNA
   <213> Homo sapiens
<220>
   <221> gene
   <222> (1)..(21)
   <223> sequence in Bst2 gene targeted by siRNA, starting from 145th base
<400> 23
   aacagcgagg cctgccggga c 21
<210> 24
   <211> 21
   <212> DNA
   <213> Homo sapiens
<220>
   <221> gene
   <222> (1)..(21)
   <223> sequence in Bst2 gene targeted by sIRNA, starting from 193th base
<400> 24
   aatgtcaccc atctcctgca a 21
<210> 25
   <211> 21
   <212> DNA
   <213> Homo sapiens
<220>
   <221> gene
   <222> (1)..(21)
   <223> sequence in Bst2 gene targeted by siRNA, starting from 212th base
<400> 25
   aacaagagct gaccgaggcc c 21
<210> 26
   <211> 21
   <212> DNA
   <213> Homo sapiens
<220>
   <221> gene
   <222> (1)..(21)
   <223> sequence in Bst2 gene targeted by siRNA, starting from 215th base
<400> 26
   aagagctgac cgaggcccag a 21
<210> 27
   <211> 21
   <212> DNA
   <213> Homo sapiens
<220>
   <221> gene
   <222> (1)..(21)
   <223> sequence in Bst2 gene targeted by siRNA, starting from 235th base
<400> 27
   aagggctttc aggatgtgga g 21
<210> 28
   <211> 21
   <212> DNA
   <213> Homo sapiens
<220>
   <221> gene
   <222> (1)..(21)
   <223> sequence in Bst2 gene targeted by siRNA, starting from 274th base
<400> 28
   aaccacactg tgatggccct a 21
<210> 29
   <211> 21
   <212> DNA
   <213> Homo sapiens
<220>
   <221> gene
   <222> (1)..(21)
   <223> sequence In Bst2 gene targeted by siRNA, starting from 294th base
<400> 29
   aatggcttcc ctggatgcag a 21
<210> 30
   <211> 21
   <212> DNA
   <213> Homo sapiens
<220>
   <221> gene
   <222> (1)..(21)
   <223> sequence in Bst2 gene targeted by siRNA, starting from 316th base
<400> 30
   aaggcccaag gacaaaagaa a 21
<210> 31
   <211> 20
   <212> DNA
   <213> Homo sapiens
<220>
   <221> gene
   <222> (1)..(20)
   <223> sequence in Bst2 gene targeted by siRNA, starting from 323th base
<400> 31
   aaggacaaaa gaagtggagg 20
<210> 32
   <211> 21
   <212> DNA
   <213> Homo sapiens
<220>
   <221> gene
   <222> (1)..(21)
   <223> sequence in Bst2 gene targeted by siRNA, starting from 331th base
<400> 32
   aagaaagtgg aggagcttga g 21
<210> 33
   <211> 19
   <212> DNA
   <213> Homo sapiens
<220>
   <221> gene
   <222> (1)..(19)
   <223> sequence in Bst2 gene targeted by siRNA, starting from 352th base
<400> 33
   ggagagatca ctacattaa 19
<210> 34
   <211> 21
   <212> DNA
   <213> Homo sapiens
<220>
   <221> gene
   <222> (1)..(21)
   <223> sequence In Bst2 gene targeted by siRNA, starting from 369th base
<400> 34
   aaaccataag cttcaggacg c 21
<210> 35
   <211> 21
   <212> DNA
   <213> Homo sapiens
<220>
   <221> gene
   <222> (1)..(21)
   <223> sequence in Bst2 gene targeted by siRNA, starting from 376th base
<400> 35
   aagcttcagg acgcgtctgc a 21
<210> 36
   <211> 21
   <212> DNA
   <213> Homo sapiens
<220>
   <221> gene
   <222> (1)..(21)
   <223> sequence in Bst2 gene targeted by siRNA, starting from 414th base
<400> 36
   aagagaaaac caggtcttaa g 21
<210> 37
   <211> 20
   <212> DNA
   <213> Homo sapiens
<220>
   <221> gene
   <222> (1)..(20)
   <223> sequence in Bst2 gene targeted by sIRNA, starting from 421th base
<400> 37
   aaccaggtct taagcgtaga 20
<210> 38
   <211> 21
   <212> DNA
   <213> Homo sapiens
<220>
   <221> gene
   <222> (1)..(21)
   <223> sequence in Bst2 gene targeted by siRNA, starting from 432th base
<400> 38
   aagcgtgaga atcgcggaca a 21
<210> 39
   <211> 21
   <212> DNA
   <213> Homo sapiens
<220>
   <221> gene
   <222> (1)..(21)
   <223> sequence in Bst2 gene targeted by siRNA, starting from 441th base
<400> 39
   aatcgcggac aagaagtact a 21
<210> 40
   <211> 21
   <212> DNA
   <213> Homo sapiens
<220>
   <221> gene
   <222> (1)..(21)
   <223> sequence in Bst2 gene targeted by siRNA, starting from 451th base
<400> 40 21
   aagaagtact accccagctc c 21